(19) **Europäisches Patentamt European Patent Office Office européen des brevets**

(11) **EP 2 780 442 B1**

(12) **FASCICULE DE BREVET EUROPEEN**

(45) Date de publication et mention
de la délivrance du brevet:
**28.03.2018 Bulletin 2018/13**

(21) Numéro de dépôt: **12795531.8**

(22) Date de dépôt: **13.11.2012**

(51) Int Cl.:
*C12N 1/12* *(2006.01)*       *C12M 1/00* *(2006.01)*
*C12M 1/09* *(2006.01)*

(86) Numéro de dépôt international:
**PCT/FR2012/052610**

(87) Numéro de publication internationale:
**WO 2013/072614 (23.05.2013 Gazette 2013/21)**

(54) **ENVELOPPE DE RÉACTION POUR UN RÉACTEUR PHOTOSYNTHÉTIQUE ET RÉACTEUR PHOTOSYNTHÉTIQUE ASSOCIÉ**

REAKTIONSGEHÄUSE FÜR EINEN FOTOSYNTHETISCHEN REAKTOR UND ZUGEHÖRIGER FOTOSYNTHETISCHER REAKTOR

REACTION CASING FOR A PHOTOSYNTHETIC REACTOR AND ASSOCIATED PHOTOSYNTHETIC REACTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.11.2011 FR 1160480**

(43) Date de publication de la demande:
**24.09.2014 Bulletin 2014/39**

(73) Titulaire: **Microphyt**
**34670 Baillargues (FR)**

(72) Inventeurs:
• **MULLER-FEUGA, Arnaud**
**F-34670 Baillargues (FR)**

• **LEMAR, Michel**
**F-69360 Saint Symphorien d'Ozon (FR)**

(74) Mandataire: **Chevalier, Renaud Philippe et al Cabinet Germain & Maureau BP 6153 69466 Lyon Cedex 06 (FR)**

(56) Documents cités:
WO-A1-2010/012028      WO-A2-2009/051479
WO-A2-2011/058267      GB-A- 2 473 865
US-A- 3 955 317

**Description**

La présente invention se rapporte à une enveloppe de réaction pour un réacteur photosynthétique adapté pour la culture de microorganismes photosynthétiques, notamment d'algues, à un procédé de fabrication d'une telle enveloppe, à un réacteur photosynthétique associé et également à un procédé de culture de microorganismes photosynthétiques associé.

Elle se rapporte plus particulièrement à une enveloppe de réaction conçue pour, d'une part, flotter sur une étendue d'eau et, d'autre part, délimiter un parcours d'écoulement diphasique gaz / milieu de culture liquide entre deux ouvertures de ladite enveloppe.

La présente invention s'applique à la culture de tout organisme photosynthétique aquatique de petite taille, c'est-à-dire de toute forme de vie susceptible de développement et de photosynthèse dans un milieu de culture nutritif approprié, en présence de rayonnement solaire et de gaz riche en carbone, tel que du dioxyde de carbone, les microalgues étant les principaux représentants de cette forme de vie.

Parmi les microorganismes photosynthétiques concernés par l'invention figurent plus particulièrement les végétaux aquatiques comme par exemple les microalgues, les protonémas de mousse, les petites macroalgues et les cellules isolées de plantes multicellulaires. Ces végétaux aquatiques présentent des propriétés intéressantes dans les domaines notamment de la pharmacie, de la nutrition humaine et animale, de la dermocosmétologie, de l'énergie et de l'environnement.

Comme pour la plupart des microorganismes photosynthétiques, l'accès à cette ressource consiste essentiellement dans la culture assistée dans des réacteurs adaptés. La lumière étant leur principal substrat, le milieu de culture doit présenter une interface optique recevant un flux de lumière. La difficulté de cultiver des microorganismes photosynthétiques tient au fait qu'ils constituent eux-mêmes des obstacles au passage de la lumière qui est leur principal substrat. La croissance de la culture va donc se stabiliser lorsque la lumière ne pénétrera plus dans l'épaisseur de la culture. Ce phénomène est appelé l'auto-ombrage.

La longueur du chemin optique, ou « light pathlength », variant généralement entre quelques centimètres et quelques décimètres, permet de caractériser les différents modes de confinement, et détermine pour l'essentiel la production de biomasse par unité de temps et de surface optique (productivité surfacique en $g/m^2/j$) et la concentration de la culture (en g/L) en phase finale de croissance.

La réaction de photosynthèse s'accompagne également d'une consommation de gaz carbonique ($CO_2$) et d'une production d'oxygène ($O_2$). L'excès d'oxygène inhibe la réaction, tandis que l'absence de gaz carbonique l'interrompt par défaut de substrat à transformer. Une interface gaz/liquide doit donc être aménagée pour les transferts de masse entre ces gaz et la phase liquide.

Afin de favoriser ces échanges et d'éviter les hétérogénéités, la culture doit être le siège d'un mélange destiné à renouveler les organismes au niveau de l'interface optique susmentionnée et également au niveau de cette interface gaz/liquide.

Il est ainsi connu, notamment dans les documents GB 2 118 572 A, ES 2 193 860 A1, GB 2 331 762 A, ES 2 150 389 A1, FR 2 685 344 A1 et FR 2 875 511 A3, d'employer des réacteurs photosynthétiques clos comprenant une boucle fermée à l'intérieur de laquelle circule le milieu de culture liquide, ladite boucle fermée comprenant une canalisation de réaction pourvue de tronçons de réaction réalisés dans un matériau transparent au rayonnement lumineux, et une canalisation de fermeture assurant la liaison entre les deux extrémités opposées de la canalisation de réaction.

La canalisation de réaction des photobioréacteurs consiste généralement en des tubes transparents horizontaux, en verre ou matière plastique, d'épaisseur ou de diamètre de l'ordre de quelques centimètres, qui sont connectées bout à bout par des coudes et des collecteurs pour former ensemble une canalisation unique en forme de serpentin ou bien des épingles aller-et-retour en parallèle.

La canalisation de fermeture comprend un tube vertical dit ascendant dans lequel le milieu liquide remonte, et un tube vertical dit descendant dans lequel le milieu liquide descend notamment sous l'effet de la gravité.

Le système d'injection de gaz généralement mis en oeuvre dans les photobioréacteurs consiste en un gazosiphon, autrement appelé « gas-lift » ou dispositif d'ascenseur à gaz, c'est-à-dire en une injection de gaz à la base du tube vertical ascendant de la canalisation de fermeture ; ladite injection de gaz servant à la fois à mettre en circulation le milieu réactionnel liquide et à réaliser les échanges gaz-liquide. Le gazosiphon comporte en partie haute un réservoir de charge ou volume élargi dans lequel les vitesses de circulation plus faibles permettent la séparation gaz-liquide, et le tube vertical descendant de la canalisation de fermeture débouche dans le fond du réservoir de charge pour alimenter en liquide la canalisation de réaction.

Les photobioréacteurs susmentionnés appliquent le principe selon lequel la réaction n'a lieu que dans la phase liquide, autrement dit ces photobioréacteurs cherchent à minimiser le volume de gaz injecté dans le réacteur pour ne pas diminuer d'autant le volume du milieu de culture liquide, dans un souci de ne pas faire diminuer la production. Ainsi, dans ces photobioréacteurs, l'extraction d'oxygène est souvent réalisée par le moyen d'un tube ascendant vertical de fermeture défini ci-dessus ; ledit tube ascendant vertical formant une colonne à bulles d'air débouchant dans le réservoir de charge recevant le milieu de culture liquide, et comportant une injection de gaz en partie basse, opportunément de l'air enrichi de $CO_2$. Comme décrit ci-dessus, les deux fonctions de circulation et de transfert gazeux sont confondues au sein de cet unique dispositif, appelé gazosiphon, qui crée une circulation verticale ascendante par échange de quantité de mouvement entre la masse liquide et les bulles de gaz résultant de l'injection. L'oxygène photosynthétique en sursaturation dans le liquide passe dans la phase gazeuse par balayage à l'air, tandis que le $CO_2$ passe en solution. Ces fonctions de dégazage et de carbonatation sont indispensables et interviennent simultanément et de façon indissociable au niveau de cet unique dispositif dans lequel la culture doit passer selon une fréquence élevée pour éviter une augmentation délétère de la teneur en oxygène dissous.

Les gazosiphons présentent l'inconvénient de générer des bulles de gaz qui remontent dans le tube ascendant vertical de la canalisation de fermeture des photobioréacteurs. La demanderesse a en effet observé le rôle délétère de ces bulles pour la culture de microorganismes dans les photobioréacteurs :

- d'une part, les bulles sollicitent mécaniquement les microalgues et peuvent nuire à des microorganismes fragiles ; et
- d'autre part, les bulles captent par effet tensio-actif les corps qui présentent des propriétés surfactantes, et notamment les molécules organiques, débris cellulaires et les produits d'excrétion des cellules vivantes. Ces substances, normalement dispersées dans le milieu en l'absence de bulles, sont ainsi rassemblées sous forme d'agrégats à la surface libre du réservoir de charge lorsque les bulles éclatent. Les bactéries et champignons qui ne pourraient se développer en raison de la forte dilution de ces corps organiques trouvent alors des substrats concentrés favorables à leur développement.

L'un des buts de la présente invention est d'éviter, ou du moins limiter, la formation des bulles pour :

- contenir le développement bactérien et fongique, par exemple pour rester compatible avec les normes sanitaires classiquement imposées dans la culture de microorganismes ; et pour
- limiter les sollicitations mécaniques dans le milieu de culture liquide, et ainsi permettre la culture de certains microorganismes fragiles qui étaient jusqu'à là exclus d'une telle culture en réacteur.

Dans une réalisation alternative au gazosiphon, la désoxygénation du milieu de culture liquide circulant dans le photobioréacteur est obtenue en faisant chuter gravitationnellement le milieu liquide dans un récipient à niveau constant. Le milieu de culture liquide est ici mis en circulation par un moyen de pompage, notamment du type pompe centrifuge, disposé dans la canalisation de réaction conçu pour non seulement compenser les pertes de charge dans la canalisation mais aussi élever la culture de la hauteur de la chute.

Bien que moins générateur de bulles, ce dispositif avec pompe centrifuge est au moins aussi mécaniquement dommageable pour les microorganismes que le gazosiphon. En effet, pour vaincre les pertes de charge, il y a génération à chaque passage au droit du moyen de pompage d'efforts mécaniques qui peuvent contrarier la croissance des microorganismes et provoquer des mortalités au sein de la culture. Les performances de production s'en trouvent alors altérées, parfois de façon rédhibitoire.

De manière générale, les cisaillements créent des tensions pouvant altérer l'intégrité cellulaire avec déchirement de la paroi des microorganismes et épanchement du cytosol, et les accélérations altèrent la structure de la cellule par augmentation du champ gravitationnel.

En outre, la Demanderesse a observé que le rendement en culture des photobioréacteurs équipés de gazosiphon ou de pompe centrifuge était limité du fait notamment des sollicitations mécaniques imposées aux organismes photosynthétiques. En effet, la Demanderesse a établi que ces sollicitations découlent en grande partie des phénomènes impliqués dans le transfert gaz-liquide pour améliorer son efficacité et éviter les effets inhibiteurs d'une teneur élevée en oxygène. La modélisation du transfert gaz-liquide du dioxyde de carbone destiné à la réaction et de l'oxygène qu'elle produit nécessite la détermination de la vitesse de transfert qui est caractérisée par le coefficient de transfert surfacique.

Ce coefficient de transfert surfacique est égal au produit du coefficient volumique de transfert de matière vers le liquide « $K_L$ » ($m.s^{-1}$) et de l'aire interfaciale rapportée au volume « $a$ » ($m^{-1}$), et dépend donc de la géométrie du système d'échange gaz/liquide mais aussi des propriétés physicochimiques du liquide et du gaz. Dans le cas d'un échange gaz/liquide au sein d'une colonne à bulles verticale, la surface d'échange dépend du nombre de bulles et de leur taille. La population de bulles générée par une injection de gaz dans un liquide dépend du débit d'injection, de la géométrie de l'injecteur, et de la différence de pression de part et d'autre de celui-ci.

La présente invention a notamment pour but de fournir un réacteur photosynthétique qui permette la culture de masse des microorganismes photosynthétiques, et son extension aux espèces les plus fragiles, avec un réacteur qui réponde aux problématiques suivantes :

- réduire voire éviter les sollicitations mécaniques liées en général à l'agitation et à la mise en circulation du milieu de culture et qui diminuent les performances de survie et de croissance des microorganismes photosynthétiques tels que les microalgues, et plus particulièrement les microalgues en chaînes pourvues d'appendices ;
- réduire voire éviter la production de bulles de petites dimensions susceptibles de favoriser l'agrégation des molécules organiques et le développement des microorganismes hétérotrophes à qui elles servent de substrat ;
- tout en réalisant le transfert photonique, pour délivrer le rayonnement solaire aux microorganismes photosynthétiques, le transfert de masse ou transfert gaz/liquide indispensable pour apporter le carbone et évacuer l'oxygène, et le transfert thermique, pour évacuer les calories apportées par le rayonnement et maintenir la culture à la bonne température ; et

- tout en maintenant des conditions mécaniques préservant l'intégrité des cellules et évitant les échanges avec le milieu environnant de nature à se prêter aux contaminations et aux disséminations.

Concernant les réacteurs photosynthétiques clos décrits ci-dessus, leur plus grande limitation au développement des productions d'algues vient du fait qu'ils sont destinés aux surfaces émergées de la planète, lesquelles servent prioritairement à l'urbanisation et aux cultures agricoles à vocation alimentaire, et présentent une rareté allant en s'accroissant avec la démographie humaine. Ce manque de surface limite sévèrement le développement par ces moyens des cultures de microalgues, notamment à vocation énergétique, qui devront, pour jouer un rôle significatif, occuper des surfaces considérables.

Or les étendues d'eau, comme par exemple les plans d'eau naturels et artificiels des continents et surtout les mers, couvrent les surfaces les plus importantes de la Terre et ne sont encore que faiblement mis en valeur pour leur exposition à la lumière.

L'un des buts poursuivi par l'invention est de proposer une enveloppe de réaction, pour un réacteur photosynthétique adapté pour la culture de microorganismes photosynthétiques, pouvant être déployée à la surface des plans d'eau et des mers. Pour cela, l'invention se propose d'utiliser ces étendues d'eau recevant le rayonnement solaire pour assurer, outre la ressource en eau, deux fonctions essentielles des photobioréacteurs, à savoir la sustentation horizontale de surface et la stabilité thermique.

Il est connu, de l'état de la technique, plusieurs types de réacteurs photosynthétiques déployés sur une étendue d'eau afin de répondre à cette problématique d'utilisation des étendues d'eau.

Il est ainsi connu, de la demande de brevet FR 2 621 323, de prévoir un photobioréacteur comportant une enveloppe de réaction réalisée sous la forme d'un premier ensemble de tubes, parallèles, en matière plastique souple, tel que du polyéthylène, connectés entre eux à leurs deux extrémités à l'aide de deux collecteurs respectivement amont et aval. Ce premier ensemble de tubes assure le confinement du milieu de culture liquide. Le photobioréacteur comprend en outre un second ensemble de tubes placé en-dessous du premier ensemble de tubes, où les tubes de ce second ensemble sont destinés à être gonflés à l'air comprimé pour former un support pneumatique flottant. Un tel photobioréacteur présente de nombreux inconvénients, dont les principaux sont : une enveloppe de réaction complexe et coûteuse avec une succession de tubes et de collecteurs amont et aval qui alourdissent l'enveloppe de réaction et une structure complexe destinée à assurer la flottaison de l'enveloppe de réaction sur l'étendue d'eau imposée notamment par la présence de ces collecteurs.

Les documents FR 2 361 060 et FR 2 324 224 décrivent respectivement un réacteur photosynthétique comportant une enveloppe de réaction réalisée sous la forme d'une série de tubes transparents reliés entre eux pour délimiter un trajet d'écoulement continu, en forme de serpentin, pour le milieu de culture liquide. Ces tubes sont logés dans un bâti pour former une structure flottante comportant des récipients de flottaison. Un tel réacteur présente de nombreux inconvénients, dont les principaux sont : une enveloppe de réaction complexe et coûteuse avec une succession de tubes raccordés entre eux à leurs extrémités, ces tubes nécessitant une structure complexe destinée à assurer la flottaison de l'ensemble.

Le document WO 2009/051479 A2 décrit un photobioréacteur comportant une enveloppe de réaction réalisée sous la forme d'une série de tubes transparents reliés entre eux par des pièces de couplage pour délimiter un trajet d'écoulement continu, en forme de serpentin, pour le milieu de culture liquide. Pour assurer la flottaison de ces tubes, le photobioréacteur comprend des flotteurs attachés sur les tubes. Un tel photobioréacteur présente de nombreux inconvénients, dont les principaux sont : une enveloppe de réaction complexe et coûteuse avec une succession de tubes raccordés entre eux à leurs extrémités par des pièces de couplage, ces tubes nécessitant l'ajout de flotteurs destinés à assurer la flottaison de l'ensemble.

Le document WO 2008/134010 A2 décrit un photobioréacteur pourvu d'une enveloppe de réaction réalisée sous la forme d'un tube en matériau flexible et transparent réalisant le confinement des volumes liquide et gazeux, et de flotteurs disposés sur les côtés du tube pour assurer la flottaison de l'ensemble. Le déploiement du volume de confinement est obtenu au moyen de raidisseurs et d'entretoises et la circulation diphasique gaz/liquide se fait dans un seul sens. Dans ce photobioréacteur, le tube doit être relié à ses deux extrémités à d'autres installations assurant la mise en mouvement et la fermeture de la boucle du liquide réactionnel.

Le document WO 2009/090549 A2 décrit un photobioréacteur pourvu d'une enveloppe de réaction réalisée sous la forme d'une poche tubulaire en matériau flexible et transparent. Dans ce photobioréacteur, l'apport en gaz ($CO_2$) au milieu de culture liquide peut se faire par diffusion passive de gaz sur une large superficie du milieu liquide, par injection de bulles de gaz, notamment en partie basse de la poche de réaction, avec tous les inconvénients mentionnés ci-dessus relatifs à la production de bulles.

Les réacteurs décrits dans les documents FR 2 621 323, FR 2 361 060, FR 2 324 224, WO 2009/051479 A2, WO 2008/134010 A2 et WO 2009/090549 A2 susmentionnés, présentent en outre un inconvénient supplémentaire commun : le nettoyage de l'enveloppe de réaction, à l'intérieur et à l'extérieur, est particulièrement complexe, et nécessite un démontage au moins partiel de l'enveloppe, sachant que le développement de salissures ou de biofilms sur les parois interne ou externe de l'enveloppe de réaction nuit à la transparence de l'enveloppe réactionnelle et donc au rendement de production des organismes photosynthétiques.

L'état de la technique peut également être illustré par l'enseignement du document GB 2 473 865 qui divulgue une enveloppe pour la culture d'algues employant deux membranes transparentes hermétiquement liées entre elles selon des lignes de jonction délimitant des cellules gonflables adjacentes et des coudes de jonction joignant deux à deux les cellules pour définir un parcours d'écoulement de forme générale sinueuse.

Il est également connu de WO 2010/012028 d'utiliser des tubes de film de polymère transparents ou translucides larges et plats qui sont disposés en séries parallèles sur un niveau plat.

Le document US 3 955 317 divulgue quant à lui une enveloppe de réaction en forme de radeau flottant et composée de tubes transparents jointifs pour définir un parcours sinueux, avec des ouvertures d'entrée et de sortie.

Afin de répondre en tout ou partie aux inconvénients et problématiques soulevés ci-dessus, la présente invention propose à cet effet une enveloppe de réaction pour un réacteur photosynthétique adapté pour la culture de microorganismes photosynthétiques, notamment d'algues, ladite enveloppe de réaction étant conforme à la revendication 1.

Avec cette enveloppe, le parcours d'écoulement diphasique présente une forme générale sinueuse ou en serpentin, et s'effectue, entre la première ouverture et la seconde ouverture, dans la succession de cellules et de coudes de jonction ; la longueur dudit parcours dépendant principalement de la longueur des cellules, les coudes de jonction servant essentiellement à attribuer au parcours cette forme sinueuse pour limiter la longueur finale de l'enveloppe.

Ainsi, cette enveloppe permet la production de microorganismes photosynthétiques, notamment de microalgues, par culture monoclonale en conditions contrôlées, pouvant être déployée à la surface d'une étendue d'eau (plan d'eau ou mer). Cette enveloppe devrait ainsi contribuer à la mise en valeur de ces étendues d'eau qui présentent les surfaces les plus abondantes et les moins mises en valeur de la planète, pour la production des microorganismes photosynthétiques.

En effet, cette enveloppe se propose d'exploiter plusieurs caractéristiques intrinsèques des étendues d'eau, à savoir :

- l'inertie thermique liée à l'importante capacité calorifique de l'eau qui, par l'échange avec le milieu de culture au travers de la gaine externe, permet de maintenir la température à des niveaux proches de l'optimum des microorganismes photosynthétiques cultivés,

- la capacité de sustentation des corps présentant une densité plus faible que l'eau qui permet d'assurer le maintien hydrostatique du volume de culture en surface selon l'horizontalité naturelle des plans d'eau, évitant ainsi les écoulements gravitaires et la formation de bulles indésirables ; et

- la transparence des étendues d'eau, lorsqu'ils ne reçoivent pas d'apport limoneux.

L'étendue d'eau peut servir de source d'eau locale pour la culture, mais il est souhaitable de la traiter afin d'en retirer les microorganismes indésirables, ainsi que certaines substances comme un excès des minéraux.

La présente enveloppe s'applique à la culture de tout organisme photosynthétique, c'est-à-dire de toute forme de vie susceptible de développement par le moyen de la photosynthèse dans un milieu de culture nutritif approprié, en présence de rayonnement solaire et de carbone, notamment sous forme de dioxyde de carbone.

Cette enveloppe de réaction, adapté aux étendues d'eau, permet de réaliser les fonctions suivantes :

- assurer dans la durée le confinement de la culture en empêchant les échanges de matière avec le milieu environnant qui se prêtent aux contaminations et aux disséminations, et pour cela résister aux sollicitations mécaniques notamment par les courants, les vents, et l'agitation de surface ;

- assurer le transfert photonique pour délivrer le rayonnement solaire aux microorganismes en culture ;

- assurer le transfert de masse gaz/liquide indispensable pour apporter le carbone et évacuer l'oxygène de la réaction ;

- assurer le transfert thermique, pour évacuer les calories apportées par le rayonnement et maintenir la culture à la bonne température ;

- se prêter à une diminution des coûts de revient de la biomasse produite par un coût modéré des moyens mis en oeuvre dans cette enveloppe.

L'enveloppe conforme à l'invention permet d'obtenir ces résultats, et présente pour cela un confinement particulier, avec des successions de cellules reliées entre elles par des coudes de jonction et délimitant ainsi un trajet unique d'écoulement continu des milieux réactionnels liquides et gazeux, qui se prête aux accroissements d'échelle vers de grandes surfaces ; la production végétale des systèmes de production de microorganismes photosynthétiques étant, en effet, proportionnelle à cette surface selon un facteur appelé productivité surfacique, dont la valeur est de l'ordre de la dizaine de grammes de matière sèche par mètre carré et par jour.

Les deux membranes sont réalisées dans un matériau souple adapté pour permettre le gonflage, mais également pour permettre le pliage, la déformation transversale et/ou le fléchissement de l'enveloppe ; chaque membrane étant réalisée d'une feuille ou film, voire d'un assemblage de feuilles ou films.

Il est entendu par matériau souple un matériau qui se laisse déformer, plier, enrouler, fléchir sans se rompre ou se casser, tel qu'un matériau flexible ou ductile. Un tel matériau est particulièrement adapté pour l'enveloppe conforme à l'invention car il permet que :

- l'enveloppe puisse être pliée ou enroulée en tout ou partie afin d'être stockée sous une forme repliée ou enroulée, avant d'être déployée sur l'étendue d'eau par gonflage, un tel gonflage étant réalisé par remplissage au moyen du gaz et/ou du liquide avant d'établir leur circulation en aller et retour ;
- les membranes de l'enveloppe se déforment et fléchissent de sorte à ne pas rompre sous l'effet du vent et des mouvements de l'étendue d'eau ;
- les coûts de fabrication de ces enveloppes sont réduits avec l'emploi d'un matériau souple relativement économique.

L'emploi d'un matériau flexible ou extensible est à privilégier pour se prêter aux déformations au niveau des extrémités de l'enveloppe.

Selon une caractéristique, les cellules comprennent plusieurs paires de cellules, chaque paire de cellules comprenant :

- une cellule dite d'aller définissant une circulation du milieu de culture liquide d'une partie amont de l'enveloppe vers une partie avale de l'enveloppe, où l'une des cellules d'aller est en liaison fluidique avec la première ouverture disposée en partie amont de l'enveloppe ; et
- une cellule dite de retour définissant une circulation du milieu de culture liquide de ladite partie avale vers ladite partie amont, où l'une des cellules de retour est en liaison fluidique avec la seconde ouverture disposée en partie amont de l'enveloppe ;

et les coudes de jonction comprennent alternativement des coudes de jonction avale disposés en partie avale de l'enveloppe et des coudes de jonction amont disposés en partie amont de l'enveloppe pour joindre deux à deux les cellules aller et retour.

Cette caractéristique géométrique est avantageuse pour limiter l'encombrement longitudinal de l'enveloppe, tout en augmentant la longueur du parcours d'écoulement diphasique ; la longueur dudit parcours d'écoulement diphasique correspondant sensiblement à la somme des longueurs des cellules.

De manière avantageuse, les coudes de jonction sont du type gonflable et sont réalisés au moins partiellement dans un matériau souple et étanche.

Selon une autre caractéristique, chaque cellule est délimitée par deux lignes de jonction rectiligne pour définir une portion rectiligne du parcours d'écoulement, et chaque coude de jonction est délimité au moins extérieurement par une ligne de jonction courbée sensiblement à 180° et présentant deux extrémités reliées à deux lignes de jonction rectiligne délimitant deux cellules adjacentes.

Ainsi, les cellules sont sensiblement rectilignes et parallèles entre elles avec des coudes à 180°.

Dans une réalisation particulière, les extrémités de chaque ligne de jonction courbée et/ou les extrémités libres des lignes de jonction rectiligne situées à l'intérieur des coudes de jonction correspondants sont munies de moyens de renfort, notamment du type oeillet, rivet ou boulon traversant de manière étanche les deux membranes.

En effet, des concentrations de tension apparaissent du fait du gonflement de l'enveloppe, notamment aux extrémités libres des lignes de jonction rectiligne séparant deux cellules communicantes et aux extrémités des lignes de jonction courbée. Ces extrémités peuvent ainsi avantageusement être renforcées par des moyens de renfort du type oeillet, rivet ou boulon traversant les membranes, à la manière des piqures d'un capitonnage. Ces moyens de renfort sont de préférence conçus pour appliquer fortement les membranes l'une contre l'autre de telle sorte que leur assemblage reste étanche malgré leur percement.

Avantageusement, chaque ligne de jonction courbée présente soit une forme générale de demi-cercle, soit une forme générale de ligne brisée formée de plusieurs segments rectilignes.

L'avantage de ces formes est d'assurer un virage pour le parcours d'écoulement entre deux cellules, tout en répondant aux contraintes imposées par le gonflement de l'enveloppe.

Selon un premier mode de réalisation avec des coudes de jonction gonflables, l'enveloppe est réalisée à partir des seules membranes supérieure et inférieure, lesdites membranes étant hermétiquement liées entre elles selon des lignes de jonction délimitant alternativement les cellules gonflables et les coudes de jonction gonflables.

Ainsi, l'enveloppe est composée d'une seule pièce issue de l'assemblage des deux membranes.

Selon un second mode de réalisation avec des coudes de jonction gonflables, l'enveloppe est composée de trois portions distinctes, à savoir une portion amont, une portion avale et une portion centrale intercalée entre les portions amont et avale, où :

- la portion amont est composée d'un assemblage d'une peau supérieure et d'une peau inférieure hermétiquement liées entre elles selon des lignes de jonction pour délimiter à la fois des portions amont des cellules et des coudes de jonction amont, les deux ouvertures étant prévues dans cette portion amont ;
- la portion avale est composée d'un assemblage d'une peau supérieure et d'une peau inférieure hermétiquement liées entre elles selon des lignes de jonction pour délimiter à la fois des portions avale des cellules et des coudes de jonction avale ; et

- la portion centrale est composée d'un assemblage d'une peau supérieure et d'une peau inférieure hermétiquement liées entre elles selon des lignes de jonction pour délimiter uniquement des portions centrales des cellules.

En variante des coudes gonflables, les coudes de jonction sont du type rigide et sont rapportés sur les extrémités des cellules gonflables.

Dans un mode de réalisation particulier, les cellules définissent des portions rectilignes du parcours d'écoulement qui sont parallèles à une direction longitudinale de l'enveloppe et qui sont de longueurs sensiblement équivalentes.

Selon une possibilité de l'invention, les lignes de jonction sont du type ligne de soudage, de couture, de collage ou d'enclipsage entre les deux membranes.

La présente invention concerne également un réacteur photosynthétique adapté pour la culture de microorganismes photosynthétiques, notamment d'algues, comportant :

- au moins une enveloppe de réaction conforme à l'invention ;
- au moins une canalisation de fermeture assurant la liaison fluidique entre les première et seconde ouvertures de ladite enveloppe de réaction ;
- au moins un moyen de mise en circulation disposé dans ladite canalisation de fermeture et conçu pour mettre en circulation le milieu de culture liquide dans la canalisation de fermeture et dans l'enveloppe de réaction ;
- au moins un moyen d'injection de liquide disposé dans ladite canalisation de fermeture et conçu pour permettre d'injecter du liquide dans l'enveloppe de réaction ;
- au moins un moyen d'injection de gaz disposé dans ladite canalisation de fermeture et conçu pour permettre d'injecter du gaz dans l'enveloppe de réaction ;
- au moins un moyen de sortie de liquide pour récolter la culture de microorganismes photosynthétiques ; et
- au moins un moyen d'échappement de gaz disposé dans ladite canalisation de fermeture et conçu pour permettre l'échappement du gaz injecté dans l'enveloppe de réaction.

Ce réacteur peut bien entendu comporter plusieurs enveloppes de réaction avec un moyen de mise en circulation commun à toutes ces enveloppes ; la ou chaque enveloppe définissant une canalisation unique depuis et jusqu'à la canalisation de fermeture qui assure la connexion entre les deux ouvertures de l'enveloppe formant l'entrée et la sortie.

Avec un réacteur selon l'invention, le milieu de culture liquide et le gaz circulent simultanément au contact l'un de l'autre le long du parcours d'écoulement diphasique sensiblement horizontal car l'enveloppe de réaction, et donc les cellules et coudes de jonction délimitant ce parcours, flottent à la surface de l'eau qui est principalement horizontale (aux variations près induites par le vent, les vagues, les mouvements de surface, etc.), et échangent certains composants le long de leur parcours commun. Les échanges entre le milieu de culture liquide et le gaz sont proportionnels à la longueur des cellules, ce qui permet d'envisager de grands accroissements d'échelle.

Le réacteur et l'enveloppe de réaction conformes à l'invention sont ainsi spécialement conçus pour accroître l'efficacité du transfert gaz-liquide et pour diminuer les sollicitations mécaniques infligées aux organismes en culture afin d'étendre la production aux espèces fragiles.

En outre, le réacteur et l'enveloppe de réaction conformes à l'invention permettent de limiter la formation de bulles de petit diamètre et ainsi de réduire le développement des microorganismes hétérotrophes consommateurs d'oxygène. En effet, avec l'enveloppe de réaction selon l'invention, le transfert gaz/liquide ne se fait plus à l'intérieur d'une colonne à bulles verticale mais le long d'un parcours d'écoulement sensiblement horizontal dans lequel l'écoulement suit un régime du type diphasique horizontal, notamment du type écoulement stratifié ou écoulement à poches ou à bulles allongées.

Contrairement au principe mentionné ci-dessus selon lequel la réaction n'a lieu que dans la phase liquide, la Demanderesse est partie du principe selon lequel le gaz fait partie intégrante de la réaction et doit être admis dans le volume réactionnel au même titre que le liquide. En privilégiant des régimes d'écoulement diphasiques horizontaux (stratifié, à poches ou à bulles allongées), la surface d'échange entre le gaz et le liquide est étendue à la totalité du parcours dans l'enveloppe de réaction avec une production de bulles nettement moins abondantes que dans le cas de certains réacteurs de l'art antérieur, réduisant de ce fait l'effet délétère observé pour ces bulles.

En outre, dans le réacteur selon l'invention, la mise en circulation du milieu de culture liquide est assurée par un ou plusieurs moyens de mise en circulation générant des forces de cisaillement et centrifuges réduites.

De manière avantageuse, la canalisation de fermeture présente une forme générale en « U » ou en « V » et comprend :

- une portion ascendante pourvue d'une partie haute en liaison fluidique avec la première ouverture ; et
- une portion descendante pourvue d'une partie haute en liaison fluidique avec la seconde ouverture ;

la portion descendante et la portion ascendante étant pourvues de parties basses respectives en liaison fluidique, et le moyen de mise en circulation étant conçu pour mettre en circulation le milieu de culture liquide dans l'enveloppe de réaction de la première ouverture vers la seconde ouverture.

Ainsi, la forme générale en « U » ou en « V » de la canalisation de fermeture, avec une portion descendante (autrement appelée branche amont plongeante) et une portion ascendante (autrement appelée branche avale montante), est destinée à bloquer le gaz à l'amont (autrement dit au niveau de la sortie de l'enveloppe qui correspond à la seconde ouverture), afin d'éviter son recyclage et également que le gaz ne réduise les performances de la mise en circulation du liquide.

Dans une première réalisation particulière, le moyen d'injection de liquide est disposé dans la partie haute de la portion ascendante, et le moyen d'injection de gaz est disposé dans la portion ascendante, en partie haute ou en partie basse de ladite portion ascendante, et en outre :

-    soit le moyen d'échappement de gaz et le moyen de sortie de liquide sont constitués d'une conduite commune disposée dans la partie haute de la portion descendante, ladite conduite commune étant pourvue d'une extrémité libre callée à une hauteur réglable par rapport à l'étendue d'eau ;
-    soit le moyen d'échappement de gaz est constitué d'une première conduite de sortie disposée dans la partie haute de la portion descendante, l'orifice de sortie vers ladite première conduite de sortie étant éventuellement pourvu d'une fermeture à flotteur, et le moyen de sortie de liquide est constitué d'une seconde conduite de sortie disposée dans la partie basse de l'une ou l'autre des portions descendante et ascendante et pourvue d'une extrémité libre callée à une hauteur réglable par rapport à l'étendue d'eau.

Dans cette première réalisation, une circulation à co-courant est mise en oeuvre avec le liquide et le gaz entrant dans l'enveloppe par la première ouverture et sortant de l'enveloppe par la seconde ouverture, de sorte que le liquide et le gaz circulent dans l'enveloppe dans le même sens de circulation ; l'injection du liquide se faisant en aval du moyen de mise en circulation, à l'entrée de l'enveloppe qui correspond à la première ouverture.

Lorsque le moyen d'échappement de gaz et le moyen de sortie de liquide sont constitués d'une conduite commune, l'extrémité libre de cette conduite commune forme un point haut qui va établir une surpression de gonflement dans l'enveloppe pour tendre les membranes ; une telle surpression de gonflement évitant que des plis ne se forment par manque de tension des membranes de l'enveloppe, ce qui entrainerait une diminution du volume des fluides qui pourraient provoquer des à-coups par carène liquide et autres coups de bélier en cas d'agitation du plan d'eau, ce qui occasionnerait des tensions brutales des membranes pouvant les déchirer.

Lorsque le moyen d'échappement de gaz et le moyen de sortie de liquide sont constitués de deux conduites de sortie distinctes, l'extrémité libre de la seconde conduite de sortie du liquide forme un point haut qui va établir une surpression de gonflement dans l'enveloppe pour tendre les membranes et éviter les plis. La présence d'une fermeture à flotteur à l'orifice de sortie de la première conduite de sortie du gaz est avantageuse pour éviter des sorties de liquide.

Dans une seconde réalisation particulière, le moyen d'injection de liquide est disposé dans la partie haute de la portion ascendante, et le moyen d'injection de gaz est disposé dans la portion descendante, de préférence en partie haute de ladite portion descendante, et en outre le moyen d'échappement de gaz est constitué d'une première conduite de sortie disposée dans la partie haute de la portion ascendante et le moyen de sortie de liquide est constitué d'une seconde conduite de sortie disposée soit dans la partie haute de la portion descendante, soit dans la partie basse de l'une ou l'autre des portions descendante et ascendante, ladite seconde conduite de sortie étant pourvue d'une extrémité libre callée à une hauteur réglable par rapport à l'étendue d'eau.

Dans cette seconde réalisation, une circulation à contre-courant est mise en oeuvre avec le liquide entrant dans l'enveloppe par la première ouverture et sortant de l'enveloppe par la seconde ouverture et avec le gaz entrant dans l'enveloppe par la seconde ouverture et sortant de l'enveloppe par la première ouverture, de sorte que le liquide et le gaz circulent dans l'enveloppe dans des sens de circulation opposés ; l'injection du liquide se faisant à l'entrée de l'enveloppe, en aval du moyen de mise en circulation.

Selon une première possibilité de l'invention, le moyen de mise en circulation est constitué d'un dispositif d'ascenseur à gaz et comprend une conduite d'injection de gaz disposé dans la partie basse de la portion ascendante, ladite conduite d'injection de gaz constituant également le moyen d'injection de gaz ; la mise en oeuvre de ce dispositif d'ascenseur à gaz n'étant envisageable qu'avec une circulation à co-courant.

Ce dispositif d'ascenseur à gaz permet d'éviter le recours à des systèmes mécaniques susceptibles de cisaillement pour les microorganismes. Cependant, le débit gazeux lié à la mise en circulation ne correspond pas toujours à celui nécessaire aux échanges gaz-liquide, ce qui peut se traduire par des fonctionnements erratiques.

Selon une seconde possibilité de l'invention, le moyen de mise en circulation est constitué d'un dispositif mécanique de mise en circulation, notamment du type hélice entraînée en rotation par un moteur ou pompe centrifuge, ledit dispositif mécanique de mise en circulation étant disposé dans la portion ascendante.

Pour dissocier la fonction de mise en circulation de celle d'injection de gaz pour les échanges de masse gaz-liquide, et ainsi maîtriser le débit gazeux nécessaire à ces échanges, il est avantageux d'employer un dispositif mécanique de mise en circulation, qui de préférence admettent le passage de corps de nettoyage circulant dans le réacteur.

Dans le cas d'une circulation à co-courant avec ce dispositif mécanique, le moyen d'injection de gaz est constitué d'une conduite d'injection de gaz disposée dans la partie haute de la portion ascendante.

L'invention se rapporte également à un procédé de culture de microorganismes photosynthétiques, notamment d'algues, utilisant un réacteur conforme à l'invention et comprenant les étapes suivantes :

- injection d'un milieu de culture liquide dans l'enveloppe de réaction selon un débit contrôlé avec le moyen d'injection de liquide ;
- injection d'un gaz dans l'enveloppe de réaction selon un débit contrôlé avec le moyen d'injection de gaz ;
- mise en circulation du milieu de culture liquide avec le moyen de mise en circulation ;
- contrôle du moyen de mise en circulation et du moyen d'injection de gaz pour établir dans l'enveloppe de réaction un régime d'écoulement diphasique gaz / milieu de culture liquide, notamment du type écoulement stratifié ou écoulement à poches ou à bulles allongées.

Selon une caractéristique, l'étape de contrôle comprend une étape de contrôle de la vitesse de circulation du liquide dans l'enveloppe de réaction entre environ 0,1 et 1,0 m/s, et une étape de contrôle de la vitesse de circulation du gaz dans l'enveloppe de réaction entre environ 0,5 et 2,0 m/s.

Cette caractéristique est avantageuse pour éviter que n'apparaissent des zones de décantation et d'autres zones d'accélération susceptibles de créer des hétérogénéités non souhaitables le long du parcours.

Selon une autre caractéristique, le moyen de mise en circulation comprend une hélice entraînée en rotation par un moteur, et la vitesse de rotation de l'hélice est inférieure à environ 1000 tours par minute, préférentiellement inférieure à environ 100 tours par minute.

Selon une caractéristique, l'injection du milieu de culture liquide et de gaz dans l'enveloppe de réaction est effectuée pour gonfler et déployer l'enveloppe sur la surface de l'étendue d'eau.

L'invention se rapporte aussi à un procédé de fabrication d'une enveloppe de réaction conforme à l'invention, comprenant les étapes suivantes :

- fournir une membrane supérieure et une membrane inférieure réalisées au moins partiellement dans un matériau souple, étanche et transparent au rayonnement lumineux ;
- relier de façon hermétique lesdites membranes selon des lignes de jonction délimitant des cellules gonflables ; et

- prévoir des coudes de jonction joignant deux à deux lesdites cellules pour définir un parcours d'écoulement de forme générale sinueuse entre une première ouverture et une seconde ouverture prévues dans ladite enveloppe.

D'autres caractéristiques et avantages de la présente invention apparaîtront à la lecture de la description détaillée ci-après, de plusieurs exemples de mise en oeuvre non limitatifs, faite en référence aux figures annexées dans lesquelles :

- la figure 1 est une vue schématique de dessus d'une enveloppe de réaction conforme à l'invention, selon un premier mode d'assemblage, avec un parcours d'écoulement composés de cinq trajets aller et retour ;
- la figure 2 est une vue schématique en coupe transversale de l'enveloppe de la figure 1 après gonflement ;
- la figure 3 est une vue schématique de dessus d'une enveloppe de réaction conforme à l'invention, selon un premier mode d'assemblage, avec un parcours d'écoulement composés de deux trajets aller et retour ;
- les figures 4a et 4b sont des vue schématiques de dessus de deux modes de réalisation de coude de jonction pour une enveloppe conforme à l'invention ;
- les figures 5a à 5c sont des vues schématiques respectivement en coupe transversale, de dessus et de côté d'un premier réacteur conforme à l'invention employant comme moyen de mise en circulation un dispositif d'ascenseur à gaz ;
- les figures 6a à 6c sont des vues schématiques respectivement en coupe transversale, de dessus et de côté d'un deuxième réacteur conforme à l'invention employant comme moyen de mise en circulation une hélice entraînée en rotation par un moteur ;
- les figures 7a à 7c sont des vues schématiques respectivement en coupe transversale, de dessus et de côté d'un troisième réacteur conforme à l'invention employant comme moyen de mise en circulation une pompe centrifuge ;
- la figure 8 est une vue schématique en coupe transversale de deux réacteurs conformes à l'invention et reliées entre eux via des canalisations de liaison ;
- les figures 9a et 9b sont des vue schématiques respectivement de dessus et de côté d'un réacteur conforme à l'invention comportant plusieurs enveloppes amarrées seulement d'un côté sur une embarcation flottante ;
- la figure 9c est une vue agrandie d'une zone de la figure 9b, illustrant la canalisation dans deux positions distinctes, à savoir une position relevée et une position abaissée ;
- la figure 10 est une vue schématique de dessus d'un réacteur conforme à l'invention comportant plusieurs enveloppes amarrées des deux côtés sur respectivement une embarcation et un système de palonnier ;

- les figures 11 a et 11b sont des vues schématiques de côté d'un réacteur conforme à l'invention comportant une enveloppe amarrée d'un côté sur une embarcation flottante, illustrant respectivement une étape de mise en place de l'enveloppe sur la canalisation de fermeture et une étape de déploiement de l'enveloppe sur l'étendue d'eau par gonflage ou remplissage ;
- la figure 12 est une vue schématique de dessus d'une enveloppe de réaction conforme à l'invention, selon un second mode d'assemblage, avant la jonction bout à bout de trois portions distinctes la composant, dont une portion centrale et deux portions d'extrémité ;
- la figure 13a est une vue schématique de dessus d'une variante de la portion centrale de l'enveloppe de la figure 12 ;
- la figure 13b est une vue schématique en coupe transversale et partielle de la variante de portion centrale de la figure 13a après gonflement ;
- les figures 14a et 14b sont des vues schématique de dessus d'une enveloppe de réaction conforme à l'invention, selon un troisième mode d'assemblage, respectivement avant et après assemblage.

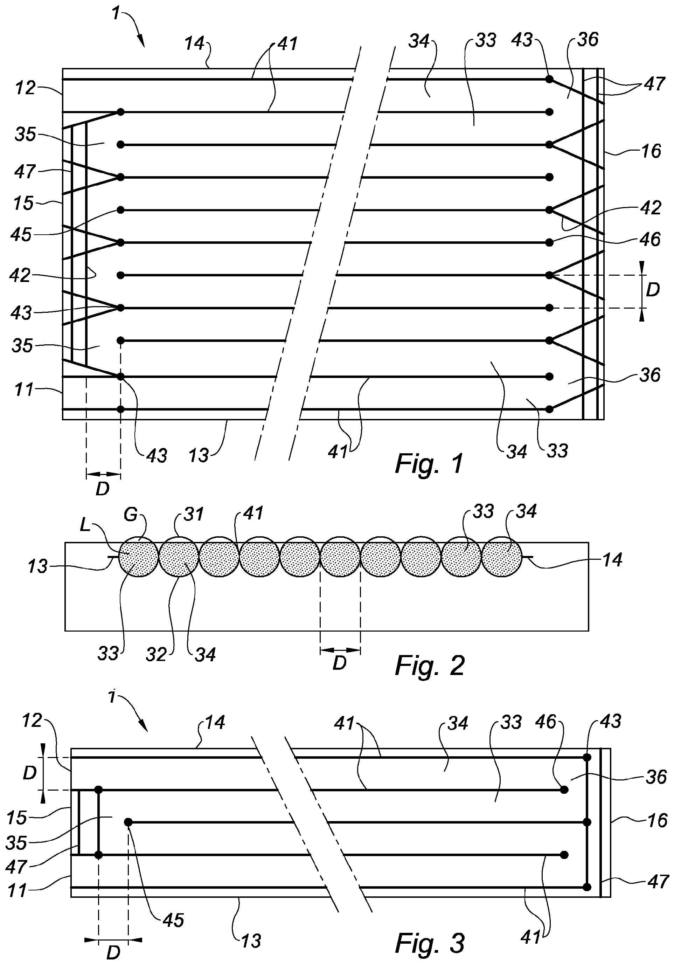

La description d'une enveloppe 1 de réaction conforme à l'invention, selon un premier mode d'assemblage, pour un réacteur photosynthétique 2, ou photobioréacteur, est faite en référence aux figures 1 à 4 ; cette enveloppe 1 étant adaptée pour la culture de microorganismes photosynthétiques, notamment d'algues, et en particulier pour la culture de microorganismes photosynthétiques fragiles aux sollicitations mécaniques.

L'enveloppe 1 est conçue pour, d'une part, flotter sur une étendue d'eau et, d'autre part, délimiter un parcours d'écoulement diphasique gaz / milieu de culture liquide entre une première ouverture 11 et une seconde ouverture 12 de l'enveloppe 1.

Cette enveloppe 1 gonflable, de forme allongée selon un axe principal longitudinal, est réalisée par l'assemblage d'une membrane supérieure 31 et d'une membrane inférieure 32 réalisées dans un matériau souple, étanche et transparent au rayonnement lumineux. L'enveloppe 1 présente deux bords longitudinaux opposés 13, 14 s'étendant dans selon l'axe principal longitudinal, et deux bords latéraux opposés, respectivement bord latéral amont 15 et bord latéral aval 16, s'étendant selon un axe secondaire latéral ; le bord latéral amont 15 étant ainsi disposé dans une partie dite amont de l'enveloppe 1 tandis que le bord latéral aval 16 est disposé dans une partie dite avale opposée. Les deux ouvertures 11, 12 sont ménagées sur le bord latéral amont 15, et ainsi dans ladite partie amont, et écartées l'une de l'autre, avec la première ouverture 11 ménagée à proximité d'un bord longitudinal 13 tandis que la seconde ouverture 12 est ménagée à proximité de l'autre bord longitudinal 14.

Ces membranes 31, 32 sont de dimensions et de forme sensiblement équivalentes, et présentent en particulier une forme générale rectangulaire avec une dimension longitudinale (ou longueur) supérieure à la dimension latérale (ou largeur). Les membranes 31, 32 présentent notamment une longueur de plusieurs dizaines de mètres de long, par exemple de l'ordre de 50 mètres.

Les membranes 31, 32 sont hermétiquement liées entre elles selon des lignes de jonction 41, 42 délimitant alternativement :

- des cellules 33, 34 gonflables ; et
- des coudes de jonction 35, 36 gonflables joignant deux à deux les cellules 33, 34 pour définir un parcours d'écoulement unique de forme générale sinueuse.

De cette manière, les cellules 33, 34 qui s'étendent selon l'axe principal longitudinal, autrement dit dans le sens de la longueur des membranes 31, 32, sont connectées bout à bout ou communiquent entre elles par les coudes de jonction 35, 36 pour former ensemble une canalisation unique en forme de serpentin.

Les cellules 33, 34 comprennent plusieurs paires de cellules, par exemple cinq paires de cellules dans l'exemple des figures 1 et 2 ou deux paires de cellules dans l'exemple de la figure 3, chaque paire de cellules comprenant :

- une cellule dite d'aller 33 définissant une circulation du milieu de culture liquide du bord latéral amont 15 vers le bord latéral aval 16 de l'enveloppe 1, où la première cellule d'aller 33 du parcours d'écoulement sinueux est en liaison fluidique avec la première ouverture 11 ; et
- une cellule dite de retour 34 définissant une circulation du milieu de culture liquide du bord latéral aval 16 vers le bord latéral amont 15, où la dernière cellule de retour 34 du parcours d'écoulement sinueux est en liaison fluidique avec la seconde ouverture 12.

Ainsi, le nombre de cellules 33, 34 est pair de telle sorte que la première ouverture 11 (entrée du milieu liquide dans l'enveloppe 1) et la seconde ouverture 12 (sortie du milieu liquide dans l'enveloppe 1) soient sur le même bord latéral amont 15. Le bord latéral amont 15 est ainsi pourvu des deux ouvertures 11, 12, avec éventuellement une ligne de jonction latérale 47 s'étendant entre les deux ouvertures 11, 12, tandis que le bord latéral aval 16 est hermétiquement fermé par au moins une ligne de jonction latérale 47 en complément des lignes de jonction 42.

Les coudes de jonction 35, 36 comprennent alternativement :

- des coudes de jonction avale 36 disposés en partie avale de l'enveloppe 1 et joignant à 180° une cellule d'aller 33 et une cellule de retour 34 d'une même paire de cellules 33, 34 ; et

- des coudes de jonction amont 35 disposés en partie amont de l'enveloppe 1 et joignant à 180° une cellule de retour 34 et une cellule d'aller 33 de deux paires de cellules 33, 34 adjacentes.

Ainsi, le parcours d'écoulement sinueux est délimité successivement, de la première ouverture 11 jusqu'à la seconde ouverture 12, par la première cellule d'aller 33, le premier coude de jonction avale 36, la première cellule de retour 34, le premier coude de jonction amont 35, la deuxième cellule d'aller 33, et ainsi de suite jusqu'au dernier coude de jonction avale 36 et enfin la dernière cellule de retour 34.

Chaque cellule 33, 34 est délimitée par deux lignes de jonction rectiligne 41 pour définir une portion rectiligne du parcours d'écoulement, et ainsi les cellules 33, 34 définissent des portions rectilignes du parcours d'écoulement qui sont parallèles à l'axe principal longitudinal de l'enveloppe 1 et qui sont de longueurs sensiblement équivalentes.

La longueur d'une enveloppe 1 dépend du plan d'eau et des approvisionnements en fluides. Avec le nombre de cellules 33, 34, cette longueur déterminera la productivité du réacteur. Par exemple, sur la base d'une productivité moyenne de 10 g/m$^2$/j, une enveloppe de 50 m de long à quatre cellules fonctionnant huit mois par an devrait pouvoir produire 80 kg de matière sèche par an.

Comme visible sur les figures 1, 3 et 4, chaque coude de jonction 35, 36 est délimité :

- extérieurement par une ligne de jonction courbée 42 sensiblement à 180°, chaque ligne de jonction courbée 42 présentant deux extrémités 43 reliées à deux lignes de jonction rectiligne 41 délimitant deux cellules 33, 34 adjacentes ; et
- intérieurement par une extrémité libre 45, 46 de la ligne de jonction rectiligne 41 concernée, à savoir une extrémité amont 45 pour un coude de jonction amont 35 et une extrémité avale 46 pour un coude de jonction avale 36.

Une fois gonflées, et comme visible sur la figure 2, les cellules 33, 34 de l'enveloppe 1 présente une forme générale tubulaire de section circulaire avec un diamètre D.

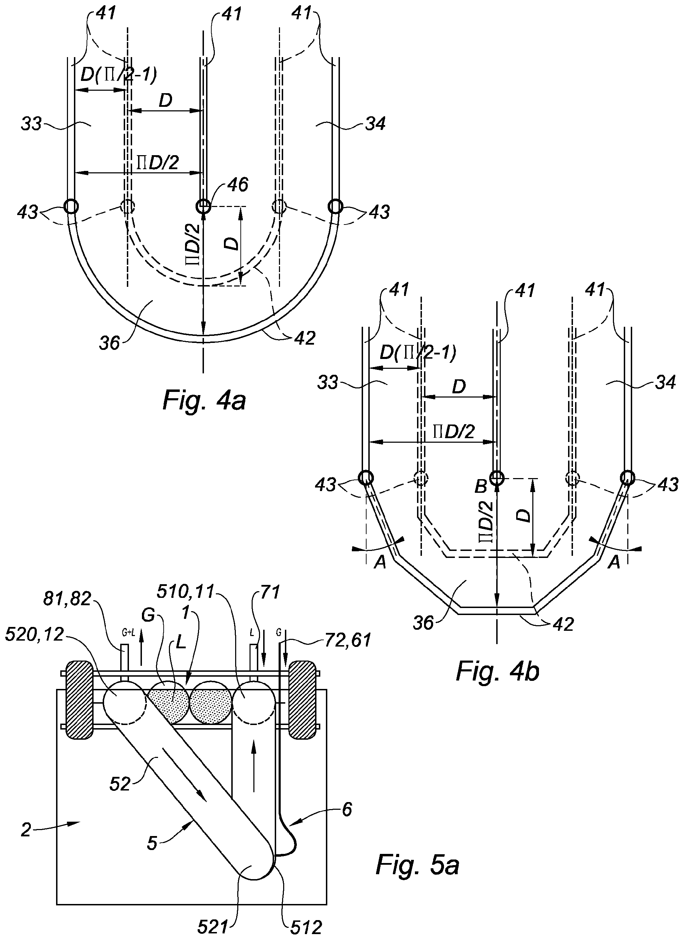

Les figures 4a et 4b illustrent :

- en traits interrompus, les lignes de jonction 41, 42 après gonflage, avec les lignes de jonction rectiligne 41 écartées l'une de l'autre d'une distance correspondant au diamètre D précité ; et
- en traits continus, les lignes de jonction 41, 42 avant gonflage, avec les lignes de jonction rectiligne 41 écartées l'une de l'autre d'une distance équivalente à πD/2 supérieure à D.

Ainsi, entre le dégonflage et le gonflage, les lignes de jonction rectiligne 41 se rapprochent d'une distance équivalente à D(π/2 - 1). De plus, les lignes de jonction courbée 42 présentent un virage à 180°.

Une difficulté dans la réalisation de ces virages, autrement dit de ces lignes de jonction courbée 42, tient au fait que ces lignes subissent, d'une part, une déformation entre la réalisation des lignes 42 et les découpes à plat et, d'autre part, une déformation tubulaire qu'ils adoptent au gonflage, des plis résultant du rétrécissement des lignes de jonction 41, 42 lors du gonflage.

Il est ainsi prévu une réalisation des lignes de jonction courbée 42 qui limite les plis à la fois pour réduire les concentrations de tension qui pourraient créer des zones de fragilité des membranes 31, 32, et pour éviter les zones de rétention de la culture difficiles d'accès aux corps de nettoyage où la biomasse pourrait s'accumuler.

Dans un premier mode de réalisation illustré sur la figure 4a, chaque ligne de jonction courbée 42 présente une forme générale de demi-cercle, autrement dit des lignes de jonction incurvées.

Dans un second mode de réalisation illustré sur les figures 1 et 4b, chaque ligne de jonction courbée 42 présente une forme générale de ligne brisée formée de plusieurs segments rectilignes, autrement dit une ligne à pan coupé. Comme visible sur la figure 4b, la courbure du virage est obtenue avec sept pans coupés, autrement dit les lignes de jonction courbée 42 sont réalisées avec cinq segments rectilignes. Les segments rectilignes d'extrémité sont destinés à éviter que deux virages consécutifs ne se télescopent au gonflage du fait du rapprochement des lignes de jonction rectiligne 41. Pour cela, ces segments rectilignes d'extrémité forment chacun un angle égal ou supérieur à A par rapport à l'axe principal longitudinal qui est indépendant du diamètre D des tubes et qui a pour valeur :

$$A = \text{Arctan} \ (1 - 2/\pi) = 19{,}97°.$$

Le segment rectiligne central est quant à lui perpendiculaire à l'axe principal longitudinal et situé à une distance au moins égale à πD/2 de l'extrémité libre 45, 46 de la ligne de jonction rectiligne 41 concerné à l'état dégonflé. Deux segments rectilignes intermédiaires joignent ce segment rectiligne central aux segments rectilignes d'extrémité respectifs pour former des pans coupés ou segments de longueurs voisines.

De même, dans le mode de réalisation de la figure 4a, la distance entre l'extrémité libre 45,46 et le sommet de la ligne de jonction courbée 42 en demi-cercle est de préférence supérieure ou égale à πD/2.

Cette largeur minimale de πD/2 pour les coudes de jonction 35, 36 dans leur plan de symétrie est avantageuse pour que la distance entre le plancher et le plafond, une fois l'enveloppe 1 gonflée, soit suffisante pour laisser passer les corps de nettoyage.

Ce second mode de réalisation n'évite pas totalement la formation de plis mais il la réduit ; de tels plis résultant du rapprochement inévitable des lignes de jonction rectiligne 41, mais l'élasticité du matériau employé pour les membranes 31, 32 permet de les limiter.

Dans un troisième mode de réalisation illustré sur la figure 3, les lignes de jonction courbée 42 sont remplacées par des lignes de jonction rectiligne orthogonales aux lignes de jonction rectiligne 41. Cependant, bien que de conception simple, une telle conformation présente l'inconvénient de former des zones de rétention de la culture dans les coins à angle droit, peu accessibles pour des corps de nettoyage.

D'autres modes de réalisation peuvent être envisagés pour limiter la formation des plis, mais cela nécessite des découpes et des lignes de jonction radiales dans les virages qui complexifient sensiblement l'assemblage des membranes 31, 32.

Il est à noter que des concentrations de tension peuvent apparaitre du fait du gonflement, notamment aux extrémités des lignes de jonction 41, 42 séparant deux cellules 33, 34 communicantes, c'est-à-dire aux extrémités 43 des lignes de jonction courbée 42 et aux extrémités libres 45, 46 des lignes de jonction rectiligne 41 situées à l'intérieur des coudes de jonction 35, 36 correspondants. Ces extrémités 43, 45, 46 sont donc avantageusement renforcées par des moyens de renfort du type oeillet, rivet ou boulon traversant les membranes 31, 32, à la manière des piqures d'un capitonnage. Ces moyens de renfort doivent appliquer fortement les membranes 31, 32 l'une contre l'autre de telle sorte que leur assemblage reste étanche malgré leur percement.

Concernant l'assemblage des deux membranes 31, 32, les lignes de jonction 41, 42 sont du type ligne de soudage, de couture, de collage ou d'enclipsage entre les deux membranes 31, 32. Ainsi, le parcours en aller et retour défini par l'enveloppe 1 est aménagé par les lignes de jonction 41, 42 au moyen de soudures, coutures, collages ou dispositifs d'enclipsage. Bien entendu, d'autres technique d'assemblage hermétiques de membranes souples peuvent être envisagées, et notamment la combinaison des techniques précitées.

Dans le cas d'un assemblage par thermo-soudage, il est envisageable de réaliser un défilement des membranes 31, 32 devant des dispositifs de chauffage équipant des machines rotatives. De manière générale, cet assemblage doit de préférence se faire dans une atmosphère microbiologiquement contrôlée de telle sorte que les enveloppes puissent être réputées stériles en sortie de fabrication.

Concernant le conditionnement d'une enveloppe 1 en sortie d'assemblage, il est envisageable d'enrouler l'enveloppe 1 autour d'un axe ou mandrin flotteur F servant à la manutention et permettant s'assurer une flottabilité de l'enveloppe 1 lors de son déroulement par gonflage sur le plan d'eau, comme décrit ultérieurement. L'enroulement de l'enveloppe 1 est tel que le bord latéral aval 16 est situé à l'intérieur de l'enroulement, tandis que le bord latéral amont 15 se présente en périphérie de l'enroulement afin d'assurer la connexion hermétique des deux ouvertures 11, 12 avec une canalisation de fermeture décrite ultérieurement. L'enroulement de l'enveloppe 1 qui suit donc l'assemblage commence donc par que le bord latéral aval 16 pour que le bord latéral amont 15 reste accessible en vue de sa connexion à la canalisation de fermeture. Une fois l'enveloppe 1 déroulée sur l'étendue d'eau, le bord latéral aval 16 est libre ou éventuellement maintenue tandis que le bord latéral amont 15 est connecté hermétiquement avec cette canalisation de fermeture pour boucler le circuit.

Les deux membranes 31, 32 sont réalisées dans un matériau souple, autrement dit dans un matériau adapté pour permettre le pliage, le gonflage, la déformation transversale et/ou le fléchissement des cellules 33, 34.

Concernant les membranes 31, 32, le matériau doit leur permettre de résister à :

- la tension qu'elles subissent lors du gonflement de l'enveloppe 1, et pour cela une surpression qui sera appliquée à l'intérieur de l'enveloppe 1 ne devra pas excéder une valeur à définir ;
- l'effort de traction lié au déplacement de la masse de l'étendue d'eau, celui de l'air étant négligeable ; et
- l'effort de rappel d'amarrage de l'enveloppe 1 à un support d'amarrage (embarcation flottante E décrite ultérieurement, quai ou berge).

La Demanderesse a ainsi établie une liste de matières plastiques utilisables pour la confection des membranes 31, 32, comprenant notamment le polyéthylène, le polypropylène, les polyamides (Nylon, Rylsan), les polytétrafluoréthylènes (PTFE), soit sous forme de membrane, soit sous forme de fibres tissées, soit sous forme composites de tissus calandrés ou enduits. Cette liste n'est bien entendu pas limitative et peut notamment être complétée par de nouveaux matériaux transparents apparaissant sur le marché.

La co-extrusion multicouche peut par exemple être utilisée pour confectionner ces membranes 31, 32 qui sont d'un usage courant en agriculture. Il est également avantageux de prévoir des matériaux légèrement extensibles pour se prêter aux déformations d'extrémité et à limiter la formation de plis.

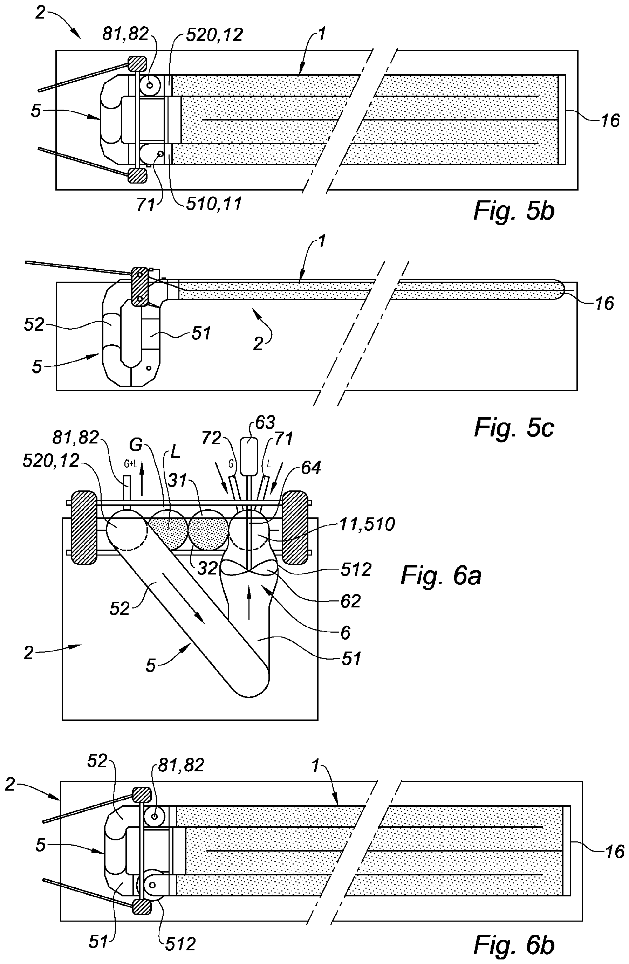

Les figures 5 à 7 illustrent des réacteurs 2 photosynthétiques conformes à l'invention et adaptés pour la culture de microorganismes photosynthétiques, notamment d'algues. Chaque réacteur 2 comprend :

- au moins une enveloppe 1 conforme à l'invention ;
- au moins une canalisation de fermeture 5 assurant la liaison fluidique entre la première 11 et la seconde ouverture 12 de l'enveloppe 1, lesdites ouvertures 11, 12 étant raccordées de façon étanche à ladite canalisation de fermeture 5 ;
- au moins un moyen de mise en circulation 6 disposé dans la canalisation de fermeture 5 et conçu pour mettre en circulation le milieu de culture liquide L dans la canalisation de fermeture 5 et dans l'enveloppe 1 ;

- au moins un moyen d'injection de liquide 71 disposé dans la canalisation de fermeture 5 et conçu pour permettre d'injecter du liquide L dans l'enveloppe 1 ;
- au moins un moyen d'injection de gaz 72 disposé dans la canalisation de fermeture 5 et conçu pour permettre d'injecter du gaz G dans l'enveloppe 1;
- au moins un moyen de sortie 81 de liquide pour récolter la culture de microorganismes photosynthétiques ;
- au moins un moyen d'échappement de gaz 82 disposé dans la canalisation de fermeture 5et conçu pour permettre l'échappement du gaz G injecté dans l'enveloppe 1.

Le réacteur 2 peut comprendre deux moyens d'injection de liquide distincts permettant d'injecter respectivement le milieu de culture liquide et l'inoculum dans le réacteur 2. Ces moyens d'injection peuvent se présenter sous la forme de ports d'injection permettant une connexion à une source avec contrôle de l'asepsie.

Le réacteur 2 peut également comprendre :

- un ou plusieurs capteurs (non illustrés) disposés sur la canalisation de fermeture 5 et adaptés pour fournir les signaux nécessaires au contrôle de la réaction, notamment des signaux représentatifs des paramètres physiques, chimiques, ou biologiques de la qualité de la culture, tels que la température, le pH, le taux d'oxygène dissous et la turbidité du milieu liquide, etc. ces contrôles servant notamment à réguler les injections de gaz et de liquide dans le réacteur 2 ;
- des moyens de contrôle de la stérilité (non illustrés) des milieux gazeux et liquides entrant et sortant de l'espace confiné par le réacteur 2, en particulier des filtres destinés à empêcher l'accès aux contaminants ;
- des boucles de régulation (non illustrées) interposées sur le circuit de la culture et destinées à réguler les principaux apports nutritifs de la culture, notamment, l'admission de milieu stérile par la concentration en matière sèche, le pH par l'injection de $CO_2$.

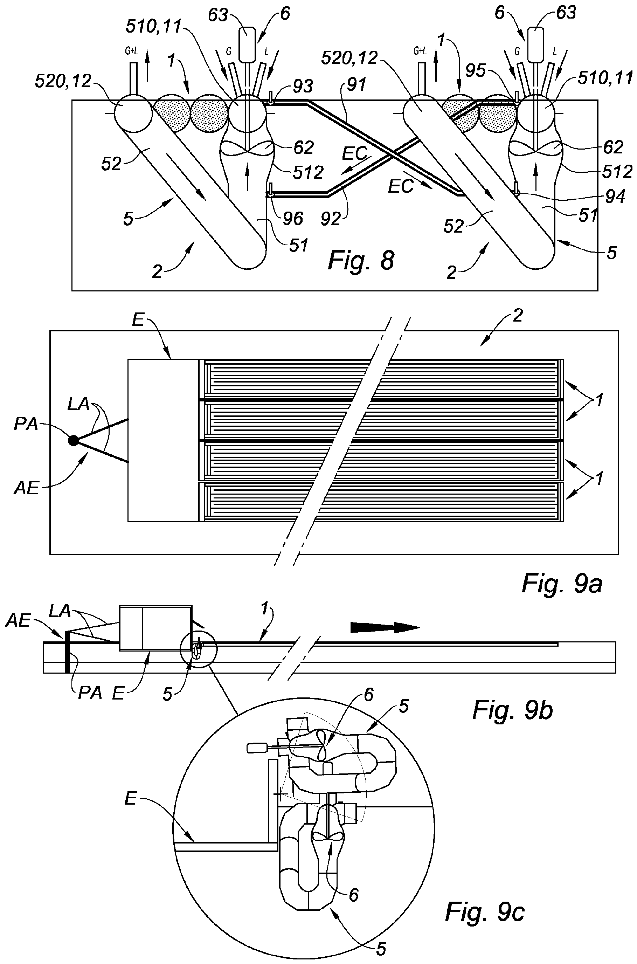

La canalisation de fermeture 5 assure la fermeture du parcours fluidique en boucle entre la première 11 et la seconde 12 ouvertures de l'enveloppe 1. La canalisation de fermeture 5 est réalisée dans une matière pouvant être non transparente au rayonnement solaire et/ou peut être disposée à l'abri de la lumière à l'intérieur d'un local fermé ou d'une embarcation E fermée comme visible notamment sur les figures 9 à 11.

La canalisation de fermeture 5 présente une forme générale en « U » ou en « V » et comprend :

- une portion ascendante 51 pourvue d'une partie ou extrémité haute 510 en liaison fluidique avec la première ouverture 11 ; et
- une portion descendante 52 pourvue d'une partie ou extrémité haute 520 en liaison fluidique avec la seconde ouverture 12.

La portion ascendante 51 et la portion descendante 52 sont pourvues de parties ou extrémités basses respectives 511, 512 en liaison fluidique. Cette forme générale en « U » ou en « V » de la canalisation de fermeture 5 est destinée à bloquer le gaz à l'amont de la canalisation de fermeture 5, c'est-à-dire en sortie de l'enveloppe 1 au niveau de la seconde ouverture 12, afin d'une part éviter son recyclage et, d'autre part, éviter qu'il ne réduise les performances de la mise en circulation du liquide.

La portion ascendante 51 et la portion descendante 52 sont de préférence tubulaires avec un diamètre interne sensiblement équivalent au diamètre D. Le moyen d'injection de liquide 71 est disposé dans la partie haute 510 de la portion ascendante 51, à l'entrée de l'enveloppe 1 au niveau de la première ouverture 11.

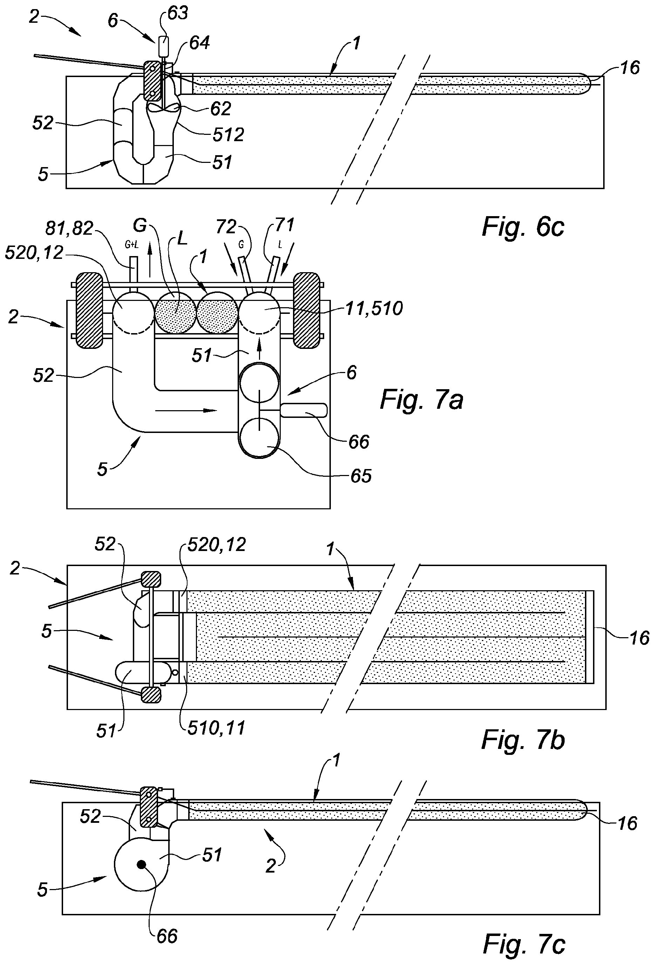

Dans les modes de réalisation des figures 6 et 7, le moyen d'injection de gaz 72 est disposé dans la partie haute 510 de la portion ascendante 51, à l'entrée de l'enveloppe 1 au niveau de la première ouverture 11.

Dans les modes de réalisation des figures 5 à 7, le moyen d'échappement de gaz 82 et le moyen de sortie de liquide 81 sont constitués d'une conduite commune 81, 82 disposée dans la partie haute 520 de la portion descendante 52, cette conduite commune 81, 82 étant pourvue d'une extrémité libre callée à une hauteur réglable par rapport à l'étendue d'eau ; cette hauteur H, visible sur les figures 6a et 7a, déterminant la surpression de gonflement de l'enveloppe 1 et pouvant être réglée pour stabiliser l'écoulement interne en fonction de l'agitation du plan d'eau. Cette hauteur H est de l'ordre de quelques centimètres et peut dépasser plusieurs décimètres pour des enveloppes de grande longueur.

Dans ce mode de réalisation, la conduite commune 81, 82 est placée dans la partie haute 520 de la portion descendante 52, juste avant la portion descendante 52 qui plonge vers le bas, pour pouvoir évacuer le gaz bloqué dans cette portion descendante 52.

Dans un mode de réalisation non illustré, le moyen d'échappement de gaz 82 est constitué d'une première conduite de sortie disposée dans la partie haute 520 de la portion descendante 52, et le moyen de sortie de liquide 81 est constitué d'une seconde conduite de sortie disposée dans la partie basse 511, 521 de l'une ou l'autre des portions descendante 51 et ascendante 52 pour ne prendre que du liquide L. L'emplacement en partie basse de cette seconde conduite de sortie 81, où se fait la récolte de la culture, est choisi pour minimiser l'influence de l'injection de milieux liquide qui aurait pour effet de la diluer.

Cette seconde conduite de sortie 81 est avantageusement pourvue d'une extrémité libre callée à une hauteur réglable par rapport à l'étendue d'eau, pour établir la surpression de gonflement. Dans ce mode de réalisation, l'orifice de sortie vers la première conduite de sortie 82 est de préférence pourvue d'une fermeture à flotteur destinée à empêcher le passage du liquide.

Ainsi, la sortie du volume de liquide L en excédent dans le réacteur 2 se fait par ce déversoir constitué par la conduite commune 81, 82 en communication avec la canalisation de fermeture 5. Le point de communication de cette conduite commune 81, 82 avec la canalisation de fermeture 5 est placé en sortie de l'enveloppe 1, à l'amont du ou des moyen d'injection de liquide 71 stérile, comme illustré sur les figures 5a, 6a et 7a, les flèches illustrant sur les figures le sens de circulation du milieu liquide L. C'est en effet à la sortie de l'enveloppe 1 que se fait la récolte de la culture et il faut éviter les courts circuits qui la dilueraient.

Cette conduite commune 81, 82, formant déversoir, constitue une rupture du confinement du milieu de culture liquide L. Afin d'éviter les rétro-contaminations de la culture en cours, la conduite commune 81, 82 peut utilement avoir une longueur de plusieurs mètres, avant de déboucher dans une cuve de récolte, et être maintenue stérile par des nettoyages périodiques.

Comme introduit ci-dessus, il faut éviter que des plis n'apparaissent, avec une diminution sensible du volume réactionnel. En effet, de tels plis pourraient provoquer des à-coups et autres coups de bélier en cas d'agitation de l'étendue d'eau, ce qui occasionnerait des tensions brutales du matériau souple de l'enveloppe 1 susceptible de provoquer son déchirement. Pour cela, l'enveloppe 1 doit être tendue en permanence grâce à une surpression de gonflement ; un contrôle de cette surpression devant permettre de maintenir l'enveloppe 1 à un niveau nominal compatible avec l'agitation de l'étendue d'eau et le bon fonctionnement de l'ensemble.

Ainsi, un procédé de culture de microorganismes photosynthétiques employant une telle enveloppe 1 comprend une étape de mise en pression de l'enveloppe 1 consistant à créer une surpression de gonflement dans cette enveloppe 1.

La surpression de gonflement de l'enveloppe 1 détermine, comme décrit ci-dessus, sa raideur, autrement dit sa résistance à la déformation liée à l'agitation du plan d'eau, et l'influence de cette dernière sur l'écoulement interne diphasique du gaz G et du liquide L ; cette surpression étant égale à la somme des pressions du gaz et du liquide dans le volume de l'enveloppe 1.

Le contrôle de la surpression de gonflement a également pour objet de déceler des fuites. La sortie du volume gazeux en excédent se fait par le moyen d'échappement de gaz 82 décrit ci-dessus et pourvu d'un orifice aménagé en partie haute 520 de la portion descendante 52. Le gaz est par exemple canalisé jusqu'à un filtre qui permet d'éviter la rétro-contamination du réacteur avant d'être relâché à l'atmosphère ou recyclé. Un contrôle du débit de sortie du gaz peut être exercé, à l'aide d'un moyen de contrôle du débit gazeux, tel qu'une vanne à pointeau, afin de régler la hauteur du ciel (niveau du liquide ou de l'interface gaz/liquide) dans l'enveloppe 1.

Dans les modes de réalisation décrits ci-dessus et illustrés sur les figures 5 à 7, une circulation à co-courant est mise en oeuvre avec le liquide et le gaz entrant dans l'enveloppe 1 par la première ouverture 11 1 par la seconde ouverture 12, de sorte que le liquide et le gaz circulent dans l'enveloppe 1 dans le même sens de circulation.

Dans des modes de réalisation non illustrés, il est envisageable de prévoir une circulation à contre-courant qui est mise en oeuvre avec le liquide entrant dans l'enveloppe 1 par la première ouverture 11 et sortant de l'enveloppe 1 par la seconde ouverture 12 et avec le gaz entrant dans l'enveloppe 1 par la seconde ouverture 12 et sortant de l'enveloppe 1 par la première ouverture 11, de sorte que le liquide et le gaz circulent dans l'enveloppe dans des sens de circulation opposés. A cet effet, le moyen d'injection de liquide 71 reste positionné à l'entrée de l'enveloppe 1, dans la partie haute 510 de la portion ascendante 51, tandis que le moyen d'injection de gaz 72 est disposé à la sortie de l'enveloppe 1, dans la portion descendante 52(en partie haute ou basse) pour que le gaz entre dans l'enveloppe 1 par la seconde ouverture 12. En outre, le moyen de sortie 81 de liquide reste positionné en sortie de l'enveloppe 1 (soit en partie haute 520 de la portion descendante 52, soit en partie basse de la portion ascendante 51 ou de la portion descendante 52) avec toujours une extrémité libre callée à une hauteur réglable par rapport à l'étendue d'eau, tandis que le moyen d'échappement de gaz 82 est positionnée en entrée de l'enveloppe 1, dans la partie haute 510 de la portion ascendante 51.

De manière générale, le moyen d'échappement de gaz 82 est conçu pour une mise à l'atmosphère du gaz ou pour un recyclage du gaz à travers un filtre qui permet d'éviter la rétro-contamination du réacteur 2. De préférence, le moyen d'échappement de gaz 82 est conformé pour maintenir la perte de charge à travers ce filtre aussi basse que possible pour maîtriser la pression dans l'enveloppe 1.

Le moyen de mise en circulation 6 est conçu pour mettre en circulation le milieu de culture liquide L dans l'enveloppe de réaction de la première ouverture 11 vers la seconde ouverture 12, ce milieu de culture liquide L circulant ainsi dans la canalisation de fermeture 5, de la partie haute 520 de la portion descendante 52 vers la partie haute 510 de la portion ascendante 51. De préférence, le moyen de mise en circulation 6 est choisi pour générer des forces de cisaillement et centrifuges réduites. Il est cependant possible d'utiliser tous types de moyens de pompage et notamment des pompes centrifuges sans que cela ne sorte du cadre de l'invention.

Dans le premier mode de réalisation du réacteur 2 illustré sur les figures 5a à 5c, le moyen de mise en circulation 6 est constitué d'un dispositif d'ascenseur à gaz et comprend ainsi une conduite d'injection de gaz 61 disposé dans la partie basse 511 de la portion ascendante 51, cette conduite d'injection de gaz 61 constituant également le moyen d'injection de gaz 72. Dans ce cas de figure, la fonction de mise en circulation est associée directement à celle d'échange de masse gaz-liquide ; ce dispositif d'ascenseur à gaz 6, de type gazosiphon, assurant à la fois le pompage et l'injection de gaz.

Dans les deux autres modes de réalisation illustrés sur les figures 6 et 7, la fonction de mise en circulation est dissociée de celle d'échange de masse gaz-liquide qui est assurée à travers leur interface dans l'enveloppe 1 et qui s'exerce sur toute leur longueur. Pour cela, le moyen de mise en circulation 6 est constitué d'un dispositif mécanique de mise en circulation qui est disposé dans la portion ascendante 51 de la canalisation de fermeture 5. Dans ces modes de réalisation, le moyen d'injection de gaz 72 est constitué d'une conduite d'injection de gaz 72 disposée dans la partie haute 510 de la portion ascendante 51, à l'entrée de l'enveloppe 1.

Dans le deuxième mode de réalisation du réacteur 2 illustré sur les figures 6a à 6c, le dispositif mécanique de mise en circulation 6 est constitué d'une hélice 62 entraînée en rotation par un moteur rotatif 63 via un arbre de sortie 64 dudit moteur 63. Le moteur 63 est disposé à l'extérieur du réacteur 2 et l'arbre de sortie 64 traverse de façon étanche la canalisation de fermeture 5 pour déboucher à l'intérieur d'un logement 512 prévu dans la portion ascendante 51, cet arbre de sortie 64 supportant l'hélice 62 qui est ainsi mobile en rotation à l'intérieur de ce logement 512.

Avantageusement, le logement 512 de l'hélice 62 est disposé entre une zone de divergence et une zone de convergence de la canalisation de fermeture 5, afin d'assurer une continuité hydraulique sans variation brusque de vitesse, ceci dans le but de limiter les pertes de charge, les accélérations et les efforts de cisaillement subis par les microorganismes.

Selon une caractéristique avantageuse et comme illustré sur les figures 6a et 6c, le logement 512 est disposé dans une partie verticale de la portion ascendante 51, et donc l'hélice 62 présente un axe vertical de rotation, afin de permettre l'évacuation du gaz G pouvant se former dans le logement 512 et éviter ainsi les phénomènes de cavitation.

De plus, et comme visible sur la figure 6a, la disposition du moyen d'échappement 82 de gaz G en amont de l'hélice 62, conjugué à la disposition de l'hélice 62 le logement 512 en amont du moyen d'injection de gaz 72 est également avantageuse pour éviter que le gaz ne circule à travers l'hélice 62 et ne nuise à son fonctionnement.

Dans le deuxième mode de réalisation du réacteur 2 illustré sur les figures 7a à 7c, le dispositif mécanique de mise en circulation 6 est constitué d'une pompe centrifuge avec une turbine 65 entraînée en rotation par un moteur rotatif 66 ; cette turbine entrainant en rotation la masse d'eau entrant axialement dans une volute de la canalisation de fermeture 5 de sorte qu'elle s'échappe tangentiellement sous l'action de la force centrifuge. Les dimensions de la volute, des pales de la turbine ainsi que leur nombre sont choisis pour livrer passage aux corps de nettoyage décrits ci-dessous.

Le réacteur 2 peut en effet comprendre un ou plusieurs corps de nettoyage (non illustrés) conformés pour circuler le long du parcours d'écoulement, autrement dit à l'intérieur de l'enveloppe 1 et de la canalisation de fermeture 5, afin de nettoyer l'intérieur de l'enveloppe 1 et de la canalisation de fermeture 5. Pour pouvoir circuler en boucle dans le réacteur 2, le ou les corps de nettoyage sont également conformés pour passer à travers le ou les moyens de mise en circulation 6 du milieu de culture liquide, par exemple à travers les pales de l'hélice 62 ou à travers la turbine 65.

Le ou les corps de nettoyage, de préférence sphériques, présentent par exemple un diamètre inférieure ou sensiblement égal au diamètre D intérieur de la canalisation de fermeture 5 et des cellules 33, 34 pour optimiser le nettoyage des parois internes de la canalisation de fermeture 7.

La différence de vitesses entre la circulation gazeuse et la circulation liquide influe directement sur les transferts de masse gaz/liquide et doit avantageusement être maintenue au niveau le plus élevé possible. C'est la raison pour laquelle chaque corps de nettoyage ne doit pas empêcher le passage du gaz. Pour cela, chaque corps de nettoyage est conformé pour laisser passer au moins partiellement le gaz circulant à l'intérieur du réacteur 2 tout en étant adapté pour être entraîné par la circulation du milieu de culture liquide afin que le corps de nettoyage n'influe pas sur la différence de vitesses entre le gaz et le milieu liquide. A cet effet, le ou chaque corps de nettoyage est réalisé sous la forme d'une brosse, notamment sphérique, comprenant un assemblage de poils, crins, brins ou équivalents, avec une partie centrale porteuse de ces poils. Ainsi, dans l'enveloppe 1 horizontale, les poils émergés laissent passer le gaz au niveau du ciel de gaz et la partie centrale immergée et porteuse des poils présente un diamètre suffisamment grand pour constituer un obstacle au passage du liquide, de sorte que le milieu liquide entraine avec lui le corps de nettoyage.

De la même manière, le corps de nettoyage peut être réalisé sous la forme d'une sphère creuse en matière élastomère. Avantageusement, une partie substantielle de la surface de la sphère peut être percée de trous qui permettent de laisser passer le gaz. Certains corps de nettoyage peuvent également présenter une densité supérieure à celle de l'eau, afin de se tenir au contact du plancher de l'enveloppe 1 correspondant à sa partie inférieure ou basse totalement immergée.

La souplesse ou l'élasticité des corps de nettoyage permet en outre de réduire les zones de rétention de la culture qui résulteraient de l'apparition des plis dans les cellules 33, 34 et/ou dans les coudes de jonction 35, 36.

Comme visible sur les figures 2, 5a, 6a et 7a, la circulation du gaz G et du liquide L, à contre-courant ou à co-courant, se fait le long d'un parcours horizontal sensiblement rectiligne où le gaz G se rassemble dans un volume situé dans les parties supérieures ou hautes des cellules 33, 34 et des coudes de jonction 35, 36. Il se crée ainsi une interface entre le gaz G et le liquide L qui est le siège des transferts liés à la réaction de photosynthèse. La forme longitudinale de cette interface, et notamment son caractère continu ou discontinu, caractérise ce que l'on appelle le régime d'écoulement. Sans considérer l'influence de l'agitation du plan d'eau, les régimes d'écoulement dans l'enveloppe 1 sont sensiblement les mêmes que dans une canalisation horizontale de section circulaire. Ces écoulements ont été décrits sous les noms d'écoulement stratifié ou à poches ou à bulles allongées.

Concernant les écoulements diphasiques dans des conduites horizontales, des travaux ont en effet mis en évidence plusieurs régimes d'écoulement selon les conditions de vitesse, de diamètre, de température, de nature, de pression des fluides circulant, à savoir notamment :

- écoulement à bulles dispersées, dispersed bubbles flow, de typologie de Mandhane AD ; et
- écoulement à bulles allongées, ou elongated bubbles flow, de typologie de Mandhane I ;
- écoulement stratifié, ou stratified flow, avec un écoulement stratifié ondulé et avec un écoulement stratifié lisse, de typologie de Mandhane SS et SW ;
- écoulement à poches, ou slug flow, de typologie de Mandhane I ;
- écoulement annulaire, ou annularmist flow, de typologie de Mandhane AD.

Dans le cas de la présente invention, les régimes d'écoulement privilégiés se situent donc au niveau de la transition SS/I dans la typologie de Mandhane, c'est-à-dire entre le régime stratifié et le régime à poches ou à bulles allongées. Dans le régime stratifié, l'interface gaz/liquide est constituée par la surface libre, dont la largeur varie avec le niveau du liquide dans l'enveloppe 1. Dans le régime à poches ou à bulles allongées, l'interface gaz/liquide est constituée par le plancher et le plafond de la poche ou de la bulle allongée. Les transferts de masse étant proportionnels à la longueur du parcours, l'effet de celle-ci sur les performances de la réaction est réduit, ce qui permet d'envisager de grands accroissements d'échelle. Les enveloppes 1 peuvent ainsi présenter des longueurs de plusieurs centaines de mètres.

Comme visible sur les figures 9 à 11, la canalisation de fermeture 5 et le moyen de mise en circulation 6 sont fixés sur une embarcation E reposant sur l'eau ; cette embarcation E pouvant être du type flottant et former une embarcation ou barge flottante, ou pouvant être du type ponton avec des poutres ou madriers plantés dans le fond de l'étendue d'eau.

Cette embarcation E peut comprendre un espace fermé, ou espace technique, dans lequel sont disposés, à l'abri des intempéries, la canalisation de fermeture 5 et le moyen de mise en circulation 6, et une paroi ou franc-bord FB sur laquelle est fixée la canalisation de fermeture 5.

Comme visible sur les figures 9 à 11, l'embarcation E est flottante et amarrée dans le fond de l'étendue d'eau, ici peu profonde, par un amarrage AE unique de l'embarcation E, de sorte que la ou les enveloppes 1 peuvent s'aligner dans le champ de courant induit par le vent à partir de cette embarcation E oscillant librement autour de son amarrage unique AE. Cet amarrage AE comprend un point d'amarrage PA, réalisé notamment sous la forme d'une poutre ou tige plantée verticalement dans le fond de l'étendue d'eau, et des liens d'amarrage LA reliant l'embarcation E au point d'amarrage PA et laissant ladite embarcation E libre en rotation autour du point d'amarrage PA. Lorsque l'amarrage AE se fait en un point unique, il faut prévoir suffisamment d'espace pour que l'ensemble puisse tourner autour de ce point sous l'influence des courants et du vent.

Dans une première forme de réalisation visible sur les figures 9a et 9b, le bord latéral aval 16 de l'enveloppe 1 est laissé libre, ce qui permet à l'enveloppe 1 de s'aligner dans le sens du déplacement relatif de la masse d'eau qui supporte l'ensemble, et a pour effet de réduire les efforts de trainée liés au déplacement de cette masse d'eau support.

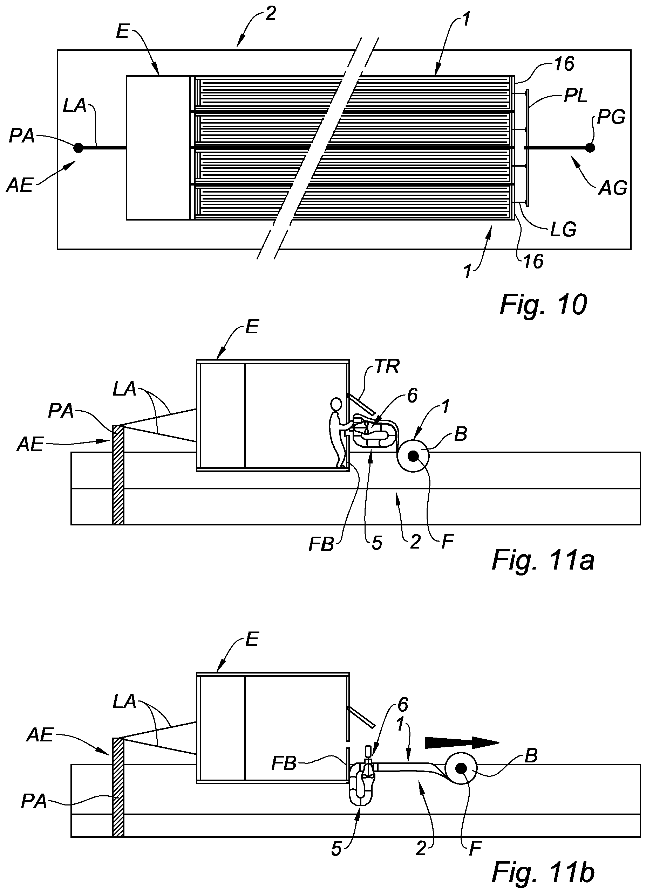

Dans une seconde forme de réalisation illustrée sur la figure 10 le bord latéral aval 16 de l'enveloppe 1 est amarré dans le fond de l'étendue d'eau, ici peu profonde, par un amarrage AG, de sorte que l'enveloppe 1 ne peut plus s'aligner dans le champ de courant induit par le vent.

Cet amarrage AG comprend des liens LG reliant les bord latéraux aval 16 des enveloppes 1 à un palonnier PL commun perpendiculaire aux cellules 33, 34, et un point d'amarrage PG, réalisé notamment sous la forme d'une poutre ou tige plantée verticalement dans le fond de l'étendue d'eau, auquel est relié le palonnier PL. L'ajustement de la longueur des liens LG et leur disposition parallèle permet de répartir les tensions d'amarrage de façon uniforme entre toutes les enveloppes 1.

Dans le cas où le plan d'eau est navigable, des moyens de signalisation diurnes et nocturnes conformes aux législations locales peuvent équiper les différents points de cet ensemble flottant, et notamment les bords latéraux aval 16 des enveloppes 1.

En outre, et comme illustré sur la figure 9c et sur les figures 11a et 11b, la canalisation de fermeture 5 peut être déplaçable en partie dans une position relevée afin de pouvoir sortir de l'eau, pour permettre notamment une mise en place de l'enveloppe 1 hors de l'eau et sous condition aseptique.

La mise en place de l'enveloppe 1 est décrite ci-après en référence aux figures 11 a et 11b. L'enveloppe 1 peut être livrées sous la forme de bobines B, où le mandrin flotteur F est positionné au centre de la bobine B pour stabiliser celle-ci en flottation sur l'étendue d'eau.

Comme visible en figure 11a, les deux ouvertures 11, 12 de l'enveloppe 1 sont hermétiquement connectées sur la canalisation de fermeture 5 ; ladite canalisation de fermeture 5 occupant avantageusement une position relevée. Cette opération de fixation de l'enveloppe 1 sur la canalisation de fermeture 5 se fait aseptiquement pour éviter d'introduire des contaminants dans le milieu de culture.

Comme visible sur la figure 11b, la canalisation de fermeture 5 est rabaissée et plongée en partie dans l'eau, puis le déploiement de l'enveloppe 1 se fait par remplissage à l'aide des milieux liquide L et gazeux G stériles ; une traction sur le mandrin flotteur F peut éventuellement aider au bon déploiement.

Une fois l'enveloppe 1 déroulée et gonflée, la circulation du milieu liquide L peut être établie dans le réacteur 2, par mise en marche du moyen de mise en circulation 6.La circulation de gaz dans l'enveloppe 1 crée une flottabilité positive répartie de façon homogène sur la longueur de l'enveloppe 1 qui les maintient en surface. L'horizontalité est assurée naturellement par la sustentation de l'étendue d'eau et par la circulation du gaz. Une trappe TR peut être prévue sur l'embarcation E afin d'isoler la partie externe du réacteur 2 de la partie interne de ce réacteur 2 une fois l'enveloppe 1 déroulée et gonflée.

Dans les modes de réalisation illustrés sur les figures, la canalisation de fermeture 5 et le moyen de mise en circulation 6 sont disposés à l'extérieur de l'embarcation E, on parle alors d'une solution dite « hors-bord ». Il est bien entendu envisageable que la canalisation de fermeture 5 présente une partie interne, disposée à l'intérieur de l'embarcation E avec le moyen de mise en circulation 6 avantageusement disposée dans cette partie interne, et une partie externe disposée à l'extérieur de l'embarcation E et connectée à l'enveloppe 1 ; on parle alors d'une solution dite « in-bord ». Dans cette solution « in-bord », il est possible de prévoir que la partie externe de la canalisation de fermeture 5 soit déplaçable par rapport à la partie interne dans une position relevée afin de pouvoir sortir celle-ci de l'eau.

Comme illustré sur la figure 8, l'invention concerne également un ensemble de réacteurs photosynthétiques comprenant au moins deux réacteurs 2 conformes à l'invention, à savoir un premier (à gauche) et un deuxième (à droite) réacteurs, et comprenant au moins une canalisation de liaison 91, 92 assurant une liaison fluidique entre le premier réacteur et le deuxième réacteur et au moins une vanne 93, 94, 95, 96 disposée sur ladite canalisation de liaison 91, 92, afin de permettre l'inoculation d'un réacteur par l'autre réacteur. Il est ainsi possible d'interconnecter deux réacteurs de telle sorte que leurs contenus soient échangés, afin de rendre possible l'inoculation d'un réacteur par un autre dont la concentration aura atteint un stade avancé.

Dans le mode de réalisation illustré sur la figure 8, l'ensemble comprend deux canalisations de liaison 91, 92 entre les deux réacteurs 2. Les canalisations de liaison 91, 92 sont pourvues, à leurs extrémités respectives, de vannes respectivement 93, 94 pour la canalisation de liaison 91 et respectivement 95, 96 pour la canalisation de liaison 92.

La première canalisation de liaison 91 relie :

- un point d'entrée disposé sur le premier réacteur 2 en aval du moyen de mise en circulation 6, tel que l'hélice 62 rotative, dans la partie haute 510 de la portion ascendante 51 ; à
- un point de sortie disposé sur le deuxième réacteur 2 en amont du moyen de mise en circulation 6 de ce deuxième réacteur 2, dans la partie basse 511 de la portion ascendante 51.

La deuxième canalisation de liaison 92 relie :

- un point d'entrée disposé sur le deuxième réacteur 2 en aval du moyen de mise en circulation 6, dans la partie haute 510 de la portion ascendante 51 ; à
- un point de sortie disposé sur le premier réacteur 2 en amont du moyen de mise en circulation 6 de ce deuxième réacteur 2, dans la partie basse 511 de la portion ascendante 51.

Les réacteurs 2 sont assemblés de façon parallèle pour constituer un ensemble productif cohérent. Afin de rendre possible l'inoculation d'un réacteur par son voisin dont la concentration en microorganismes aurait atteint un stade avancé, l'ensemble prévoit d'interconnecter ces deux réacteurs avec les canalisations de liaison 91, 92 de telle sorte que leurs contenus respectifs soient mélangés.En outre, les points de sortie des canalisations de liaison 91, 92 sont placés à l'extrémité des zones de convergence situées en amont des logements 512 correspondants pour bénéficier d'un effet Venturi.

Les vannes 93, 94, 95, 96 permettent la connexion sous asepsie des deux canalisations de liaison 91, 92 qui relient de façon croisée et symétrique les points d'entrée et les points de sortie des deux réacteurs 2 à interconnecter. Les vannes 93, 94, 95, 96 sont disposées sensiblement aux points d'entrée et de sortie des canalisations de liaison 91, 92 correspondantes.

L'utilisation d'un tel ensemble peut se faire de la manière suivante pour procéder à l'inoculation du deuxième réacteur 2 (à droite) à partir du premier réacteur 2 (à gauche) déjà en service lorsque la concentration en microorganismes a atteint le niveau d'exploitation.

Dans un premier temps, les vannes 93, 94 et leurs vis-à-vis 95, 96 sont fermées, le premier réacteur 2 est en service avec l'établissement de la circulation à l'intérieur de ce premier réacteur, et le deuxième réacteur 2 à inoculer est rempli de milieu nutritif stérile.Dans un deuxième temps, la circulation est établie à l'intérieur du deuxième réacteur 2 et les vannes 93, 94 et leurs vis-à-vis 95, 96 sont ouvertes pour établir un échange croisé entre les deux réacteurs comme illustré par les flèches EC de la figure 8. L'interconnexion entre les deux réacteurs 2 est établie entre l'amont et l'aval des moyens de mise en circulation 6 pour que la force propulsive obtenue avec ces moyens de mise en circulation 6 favorise la circulation d'échange. Après l'ouverure des vannes 93, 94 et leurs vis-à-vis 95, 96, les concentrations deviennent sensiblement égales dans les deux réacteurs 2 et il est possible de les isoler par fermeture des vannes 93, 94 et leurs vis-à-vis 95, 96. Pour réduire la durée de cet échange, une pompe (non visible) peut être interposée sur l'une et/ou l'autre des canalisations de liaison 91, 92.

Comme illustré schématiquement sur les figures 9a et 10, l'invention concerne également un réacteur 2 comportant une pluralité d'enveloppes 1 de réaction raccordées de façon parallèle, ce réacteur 2 pouvant comprendre une seule et même canalisation de fermeture (non visible) dans laquelle est disposée un unique moyen de mise en circulation commun à l'ensemble des enveloppes 1. Dans cette forme particulière de réalisation, qui consiste à interconnecter des enveloppes 1 de réaction en série ou en parallèle, l'intérêt est de mettre en commun certaines fonctionnalités, comme les moyens de mise en circulation et les moyens de régulation.

Dans une configuration en parallèle, la canalisation de fermeture comporte une conduite de collecte dans laquelle est disposé le moyen de mise en circulation, et une pluralité de conduites de distribution connectées, d'une part, à la conduite de collecte et, d'autre part, aux enveloppes 1 respectives, de sorte que le milieu liquide est collecté en sortie des enveloppes 1, passe dans le moyen de mise en circulation, puis est distribué à l'entrée des enveloppes 1.

Dans une configuration série, la seconde ouverture 12 (sortie du milieu liquide) d'une enveloppe 1 est directement connectée à la première ouverture 11 (entrée du milieu liquide) de l'enveloppe 1 suivante, et la canalisation de fermeture est disposée entre la première ouverture 11 de la première enveloppe et la seconde ouverture 12 de la dernière enveloppe.

Dans la configuration en parallèle, l'efficacité du nettoyage n'est cependant pas optimale car les corps de nettoyage sont distribués de façon aléatoire dans les enveloppes 1. C'est pourquoi on lui préfère la configuration en série où les corps de nettoyage doivent suivre un parcours unique d'enveloppe en enveloppe.

Le procédé de culture de microorganismes photosynthétiques, notamment d'algues, utilisant un réacteur 2 conforme à l'invention, comprend les étapes suivantes :

- injection d'un milieu de culture liquide L dans l'enveloppe 1 de réaction selon un débit contrôlé avec le moyen d'injection 71 de liquide ;
- injection d'un gaz G dans l'enveloppe 1 de réaction selon un débit contrôlé avec le moyen d'injection 72 de gaz ;
- mise en pression de l'enveloppe 1 consistant à créer une surpression de gonflement dans cette enveloppe 1 pour assurer la flottabilité de celle-ci et son déploiement ;
- mise en circulation du milieu de culture liquide avec le moyen de mise en circulation 6 ;
- contrôle du moyen de mise en circulation 6 et du moyen d'injection 72 de gaz pour établir dans l'enveloppe 1 de réaction un régime d'écoulement diphasique gaz / milieu de culture liquide du type écoulement stratifié ou écoulement à poches ou à bulles allongées ; et
- récupération des microorganismes photosynthétiques avec le moyen de sortie 81 de liquide.

Pendant le parcours dans l'enveloppe 1, le milieu liquide contenant les microorganismes photosynthétiques reçoit le rayonnement solaire à travers la matière transparente des membranes 31, 32 de l'enveloppe 1, échange de la chaleur avec le plan d'eau par diffusion, par mélange et par conduction à travers cette même matière, et échange des composants avec le gaz G à travers leur interface commune. La capacité de production dépend surtout de la longueur de l'enveloppe 1 et du nombre de cellules 33, 34.

De façon avantageuse, la vitesse de circulation du liquide dans l'enveloppe 1 est comprise entre environ 0,1 et 1,0 m/s.

En outre, la vitesse de circulation du gaz est établie entre environ 0,5 et 2,0 m/s, correspondant à un régime de vitesses adéquat pour les débits nécessaires à la réaction.

De façon encore avantageuse, le moyen de mise en circulation 6 comprenant une hélice 62 entraînée en rotation par un moteur 63 est piloté de sorte que la vitesse de rotation de l'hélice 62 est inférieure à environ 1000 tours par minute, afin de limiter les sollicitations mécaniques au sein du milieu de culture liquide.

L'invention concerne également un procédé de fabrication d'une enveloppe 1 selon le premier mode d'assemblage ou selon le deuxième mode d'assemblage (décrit ci-après) et comprenant les étapes suivantes :

- fournir une membrane supérieure 31 et une membrane inférieure 32 réalisées au moins partiellement dans un matériau souple, étanche et transparent au rayonnement lumineux ;
- recouvrir la membrane inférieure 32 par la membrane supérieure 31 ;
- relier hermétique les deux membranes 31, 43 selon des lignes de jonction 41, 42 délimitant alternativement des cellules 33, 34 gonflables et des coudes de jonction 35, 36 gonflables joignant deux à deux les cellules 33, 34 pour définir un parcours d'écoulement de forme générale sinueuse entre les deux ouvertures 11, 12, les deux ouvertures 11, 12 étant prévues aux extrémités de ce parcours ; et
- enroulement de l'enveloppe 1 sur un touret de manière à former une bobine B.

Dans le premier mode d'assemblage décrit ci-dessus en référence aux figures 1 à 4, l'enveloppe 1 est réalisée à partir des seules membranes 31, 32 qui sont assemblées entre elles selon les lignes de jonction, et deux réalisations sont envisageables.

Dans une première réalisation du premier mode d'assemblage de l'enveloppe 1, les deux membranes 31, 32 sont composées de deux feuilles (autrement appelée films) distinctes et sépareées l'une de l'autre avant assemblage, l'assemblage consistant ensuite à les raccorder entre elles grâce aux lignes de jonction 41, 42, 47. Dans ce mode de réalisation, la membrane inférieure 32 n'est pas nécessairement transparente au rayonnement solaire, seule la membrane supérieure 31 devant impérativement l'être.

Dans une seconde réalisation du premier mode d'assemblage, les deux membranes 31, 32 sont composées d'une seule et même feuille (autrement appelée film) qui est repliée en deux sur elle-même dans le sens de sa longueur suivant une ligne de pliage longitudinale, puis les lignes de jonction 41, 42, 47 sont réalisées pour joindre hermétiquement les deux faces de la feuille et ainsi former l'enveloppe 1 ; la ligne de pliage longitudinale pouvant éventuellement constituer une ligne de jonction 41.

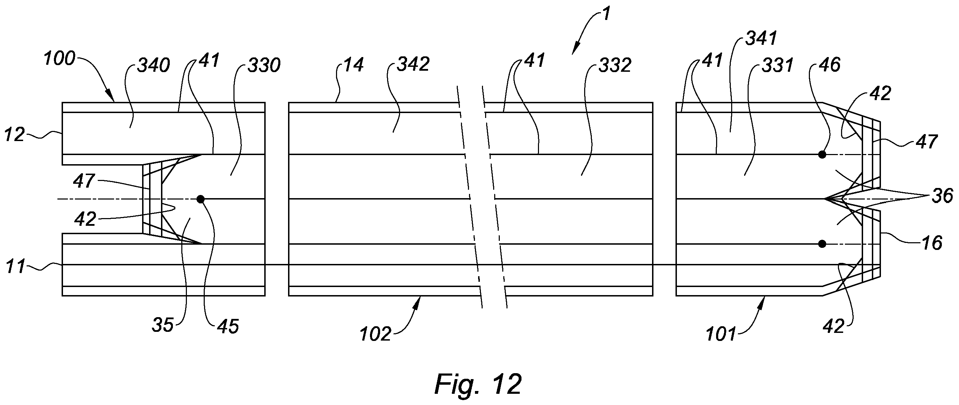

Dans un deuxième mode d'assemblage décrit en référence à la figure 12, l'enveloppe 1 est composée de trois portions distinctes, à savoir une portion amont 100, une portion avale 101 et une portion centrale 102 intercalée entre les deux portions d'extrémité 100 et 101.

La portion amont 100 est composée d'un assemblage d'une peau (autrement appelée feuille ou film) supérieure et d'une peau inférieure hermétiquement liées entre elles selon des lignes de jonction 41, 42, 47 pour délimiter à la fois des portions amont 330, 340 des cellules 33, 34 et les coudes de jonction amont 35, les deux ouvertures 11, 12 étant prévues dans cette portion amont 100.

La portion avale 101 est composée d'un assemblage d'une peau supérieure et d'une peau inférieure hermétiquement liées entre elles selon des lignes de jonction 41, 42, 47 pour délimiter à la fois des portions avale 331, 341 des cellules 33, 34 et les coudes de jonction avale 36.

La portion centrale 102 est composée d'un assemblage d'une peau supérieure et d'une peau inférieure hermétiquement liées entre elles selon des lignes de jonction 41 pour délimiter uniquement des portions centrales 332, 342 des cellules 33, 34. La portion centrale 102 présente une longueur équivalente à au moins 90% de la longueur totale de l'enveloppe 1.

Ainsi, les trois portions 100, 101, 102 sont réalisées indépendamment l'une de l'autre, puis jointes bout à bout pour former l'enveloppe 1 ; la membrane supérieure 31 de l'enveloppe 1 étant constituée de l'assemblage des peaux supérieures de ces portions 100, 101, 102, tandis que la membrane inférieure 32 de l'enveloppe 1 est constituée de l'assemblage des peaux inférieures de ces portions 100, 101, 102. La jonction bout à bout de ces trois portions 100, 101, 102 est réalisée pour que les lignes de jonction 41 des différentes portions soient alignées et continues.

L'avantage principale de cette réalisation avec trois portions est de faciliter la réalisation des coudes 35, 36 pour garantir leurs géométries. En effet, il est important de respecter la géométrie décrite ci-dessus pour les coudes 35, 36, et en particulier la largeur minimale des coudes 35, 36 dans leurs plans de symétrie respectifs, ce qui impose de maîtriser l'interruption des lignes de jonction 41 au niveau de leurs extrémités libres 45, 46. En travaillant avec des portions d'extrémité 100, 101 courtes, il est ainsi plus facile de contrôler la localisation et l'interruption des lignes de jonction 41 et garantir la géométrie souhaitée.

En outre, il est envisageable de choisir des peaux non transparentes au rayonnement solaire pour réaliser les portions d'extrémité 100 et 101, ce qui permet éventuellement de sélectionner des matériaux plus adaptés pour réaliser des lignes de jonction courbées 42 et des interruptions dans les lignes de jonction rectilignes 41. Seule la portion centrale 102 devant être réalisée dans un matériau transparent au rayonnement solaire, voire même seule la peau supérieure de la portion centrale 102 devant être réalisée dans un matériau transparent au rayonnement solaire. La portion centrale 102 peut être réalisée par assemblage de deux feuilles distinctes, ou par pliage d'une seule et même feuille.

Dans les modes de réalisation décrit ci-dessus en référence aux figures 1 à 4 et 12, chaque cellule 33, 34 est séparée de la ou des cellules adjacentes par une seule ligne de jonction 41.

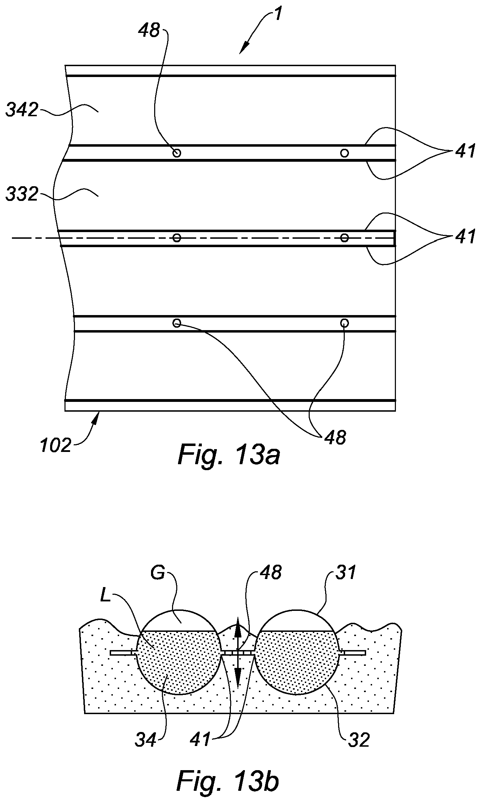

Dans une variante illustrée sur les figures 13a et 13b, chaque cellule 33, 34 est séparée de la ou des cellules adjacentes par deux lignes de jonction 41 parallèles et espacées l'une de l'autre. Ce doublement des lignes de jonction 41 entre les cellules 33, 34 peut être envisagé avec l'enveloppe 1 du premier mode d'assemblage, ou bien avec l'enveloppe 1 du second mode d'assemblage ou chaque portion 100, 101, 102 présente un dédoublement de ces lignes de jonction 41 entre les portions 330, 340, 331, 341, 332, 342 des cellules 33, 34. Dans ce cas de figure, il est envisageable de prévoir des trous 48 dans l'enveloppe 1 entre les deux lignes de jonction 41 appairées (bien évidément pas dans les cellules 33, 34), pour permette l'écoulement d'eau à travers ces trous 48 une fosi l'enveloppe 1 gonflée et flottant sur la surface de l'eau.

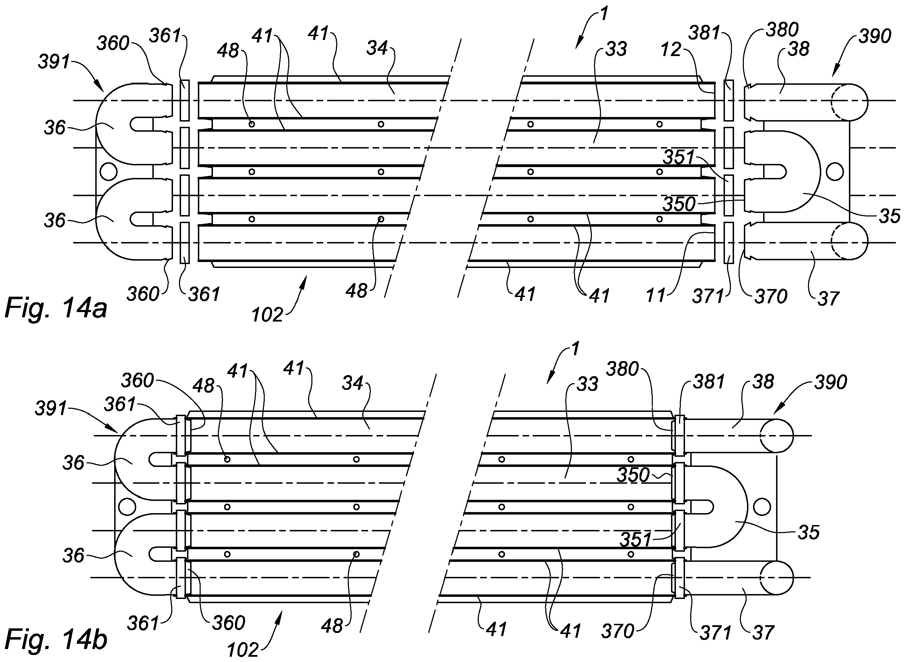

Dans un troisième mode d'assemblage décrit en référence aux figures 14a et 14b, l'enveloppe 1 comporte des coudes de jonction 35, 36 rigides qui sont rapportés de manière étanche sur les extrémités des cellules 33, 34 gonflables.

Dans le mode d'assemblage illustré sur les figures 14a et 14b, l'enveloppe 1 est composée d'une portion centrale 102 du même type que celle de la figure 13a ou de la figure 12, qui est composée d'un assemblage d'une peau supérieure et d'une peau inférieure hermétiquement liées entre elles selon des lignes de jonction 41 (soit simples soit doublées) pour les cellules 33, 34.

Pour rapporter les coudes 35, 36 rigides, ces derniers présentent de préférence des embouts 350, 360 qui sont introduits à l'intérieur des extrémités des cellules, puis des colliers de serrage 351, 361 sont serrés autour de ces embouts 350, 360 pour garantir l'étanchéité.

De même, des conduits rigides d'entrée 37 et de sortie 38 peuvent être rapportés sur les ouvertures 11, 12 de l'enveloppe 1 ; ces conduits rigides d'entrée 37 et de sortie 38 présentant de préférence des embouts 370, 380 qui sont introduits à l'intérieur des ouvertures 11, 12, puis des colliers de serrage 371, 381 sont serrés autour de ces embouts 370, 380 pour garantir l'étanchéité.

Par ailleurs, il est envisageable que les coudes de jonction avale 36 rigides soient réunis au sein d'une même pièce avale 391 rigide, et que les coudes de jonction amont 35 rigides et les conduits rigides 37, 38 soient réunis au sein d'une même pièce amont 390 rigide.

Bien entendu l'exemple de mise en oeuvre évoqué ci-dessus ne présente aucun caractère limitatif et d'autres améliorations et détails peuvent être apportés à l'enveloppe, au réacteur et aux procédés selon l'invention, sans pour autant sortir du cadre de l'invention où d'autres formes de membrane et/ou de cellule et/ou de coude de jonction et/ou de canalisation de fermeture peuvent par exemple être réalisées.

## Revendications

1. Enveloppe (1) de réaction pour un réacteur (2) photosynthétique adapté pour la culture de microorganismes photosynthétiques, notamment d'algues, ladite enveloppe (1) de réaction étant conçue pour, d'une part, flotter sur une étendue d'eau et, d'autre part, délimiter un parcours d'écoulement diphasique gaz / milieu de culture liquide entre une première et une seconde ouvertures (11, 12) de ladite enveloppe (1) de réaction, où ladite enveloppe (1) de réaction comporte, d'une part, une membrane supérieure (31) et une membrane inférieure (32) réalisées au moins partiellement dans un matériau souple, étanche et transparent au rayonnement lumineux, lesdites membranes (31, 32) étant hermétiquement liées entre elles selon des lignes de jonction (41, 42) délimitant des cellules (33, 34) gonflables adjacentes et, d'autre part, des coudes de jonction (35, 36) joignant deux à deux lesdites cellules (33, 34) pour définir ledit parcours d'écoulement de forme générale sinueuse, l'une des cellules (33) étant en liaison fluidique avec la première ouverture (11) et une autre cellule (34) étant en liaison fluidique avec la seconde ouverture (12), où les coudes de jonction (35, 36) sont du type gonflable et sont réalisées au moins partiellement dans un matériau souple et étanche, ladite enveloppe (1) de réaction étant **caractérisée en ce que** l'enveloppe (1) est composée de trois portions distinctes, à savoir une portion amont (100), une portion avale (101) et une portion centrale (102) intercalée entre les portions amont et avale (100, 101), où :

- la portion amont (100) est composée d'un assemblage d'une peau supérieure et d'une peau inférieure hermétiquement liées entre elles selon des lignes de jonction (41, 42, 47) pour délimiter à la fois des portions amont (330, 340) des cellules (33, 34) et des coudes de jonction amont (35), les deux ouvertures (11, 12) étant prévues dans cette portion amont (100) ;
- la portion avale (101) est composée d'un assemblage d'une peau supérieure et d'une peau inférieure hermétiquement liées entre elles selon des lignes de jonction (41, 42, 47) pour délimiter à la fois des portions avale (331, 341) des cellules (33, 34) et des coudes de jonction avale (36) ; et
- la portion centrale (102) est composée d'un assemblage d'une peau supérieure et d'une peau inférieure hermétiquement liées entre elles selon des lignes de jonction (41) pour délimiter uniquement des portions centrales (332, 342) des cellules (33, 34).

2. Enveloppe (1) selon la revendication 1, dans laquelle chaque cellule (33 ; 34) est délimitée par deux lignes de jonction rectiligne (41) pour définir une portion rectiligne du parcours d'écoulement, et chaque coude de jonction (35 ; 36) est délimité au moins extérieurement par une ligne de jonction courbée (42) sensiblement à 180° et présentant deux extrémités (43) reliées à deux lignes de jonction rectilignes (41) délimitant deux cellules (33, 34) adjacentes.

3. Enveloppe (1) selon la revendication 2, dans laquelle les extrémités (43) de chaque ligne de jonction courbée (42) et/ou les extrémités libres (45, 46) des lignes de jonction rectiligne (41) situées à l'intérieur des coudes de jonction (35, 36) correspondants sont munies de moyens de renfort, notamment du type oeillet, rivet ou boulon traversant de manière étanche les deux membranes.

4. Enveloppe (1) selon les revendications 2 ou 3, dans laquelle chaque ligne de jonction courbée (42) présente soit une forme générale de demi-cercle, soit une forme générale de ligne brisée formée de plusieurs segments rectilignes.

5. Réacteur (2) photosynthétique adapté pour la culture de microorganismes photosynthétiques, notamment d'algues, comportant :

- au moins une enveloppe (1) de réaction conforme à l'une quelconque des revendications précédentes ;
- au moins une canalisation de fermeture (5) assurant la liaison fluidique entre les première et seconde ouvertures (11, 12) de ladite enveloppe (1) de réaction ;
- au moins un moyen de mise en circulation (6) disposé dans ladite canalisation de fermeture (5) et conçu pour mettre en circulation le milieu de culture liquide dans la canalisation de fermeture (5) et dans l'enveloppe (1) de réaction ;
- au moins un moyen d'injection de liquide (71) disposé dans ladite canalisation de fermeture (5) et conçu pour permettre d'injecter du liquide dans l'enveloppe (1) de réaction ;
- au moins un moyen d'injection de gaz (72) disposé dans ladite canalisation de fermeture (5) et conçu pour permettre d'injecter du gaz dans l'enveloppe (1) de réaction ;
- au moins un moyen de sortie de liquide (81) pour récolter la culture de microorganismes photosynthétiques ; et
- au moins un moyen d'échappement de gaz (82) disposé dans ladite canalisation de fermeture (5) et conçu pour permettre l'échappement du gaz injecté dans l'enveloppe (1) de réaction.

6. Réacteur (2) photosynthétique selon la revendication 5, dans lequel la canalisation de fermeture (5) présente une forme générale en « U » ou en « V » et comprend :

- une portion ascendante (51) pourvue d'une partie haute (510) en liaison fluidique avec la première ouverture (11) ; et
- une portion descendante (52) pourvue d'une partie haute (520) en liaison fluidique avec la seconde ouverture (12) ;

la portion descendante (52) et la portion ascendante (51) étant pourvues de parties basses (511, 521) respectives en liaison fluidique, et le moyen de mise en circulation (6) étant conçu pour mettre en circulation le milieu de culture liquide dans l'enveloppe (1) de réaction de la première ouverture (11) vers la seconde ouverture (12).

7. Réacteur (2) photosynthétique selon la revendication 6, dans lequel le moyen d'injection de liquide (71) est disposé dans la partie haute (510) de la portion ascendante (51), et le moyen d'injection de gaz (72) est disposé dans la portion ascendante (51), en partie haute (510) ou en partie basse (511) de ladite portion ascendante (51), et dans lequel :

- soit le moyen d'échappement de gaz (82) et le moyen de sortie de liquide (81) sont constitués d'une conduite commune disposée dans la partie haute (520) de la portion descendante (52), ladite conduite commune étant pourvue d'une extrémité libre callée à une hauteur réglable par rapport à l'étendue d'eau ;
- soit le moyen d'échappement de gaz (82) est constitué d'une première conduite de sortie disposée dans la partie haute (520) de la portion descendante (52), l'orifice de sortie vers ladite première conduite de sortie (81) étant éventuellement pourvu d'une fermeture à flotteur, et le moyen de sortie de liquide (81) est constitué d'une seconde conduite de sortie disposée dans la partie basse (511 ; 521) de l'une ou l'autre des portions ascendante (51) et descendante (52) et pourvue d'une extrémité libre callée à une hauteur réglable par rapport à l'étendue d'eau.

8. Réacteur (2) photosynthétique selon la revendication 6, dans lequel le moyen d'injection de liquide (71) est disposé dans la partie haute (510) de la portion ascendante (51), et le moyen d'injection de gaz (72) est disposé dans la portion descendante (52), de préférence en partie haute (520) de ladite portion descendante (52), et dans lequel le moyen d'échappement de gaz (82) est constitué d'une première conduite de sortie disposée dans la partie haute (510) de la portion ascendante (51) et le moyen de sortie de liquide (81) est constitué d'une seconde conduite de sortie disposée soit dans la partie haute (520) de la portion descendante (52), soit dans la partie basse (511 ; 521) de l'une ou l'autre des portions ascendante (51) et descendante (52), ladite seconde conduite de sortie étant pourvue d'une extrémité libre callée à une hauteur réglable par rapport à l'étendue d'eau.

9. Réacteur (2) photosynthétique selon la revendication 7, dans lequel le moyen de mise en circulation (6) est constitué d'un dispositif d'ascenseur à gaz et comprend une conduite d'injection de gaz disposé dans la partie basse (511, 521) de la portion ascendante (51), ladite conduite d'injection de gaz constituant également le moyen d'injection de gaz (72).

10. Réacteur (2) photosynthétique selon l'une quelconque des revendications 6 à 8, dans lequel le moyen de mise en circulation (6) est constitué d'un dispositif mécanique de mise en circulation, notamment du type hélice (62) entraînée en rotation par un moteur (63) ou pompe centrifuge (65, 66), ledit dispositif mécanique de mise en circulation étant disposé dans la portion ascendante (51).

11. Procédé de culture de microorganismes photosynthétiques, notamment d'algues, utilisant un réacteur (2) conforme à l'une quelconque des revendications 5 à 10 et comprenant les étapes suivantes :

- injection d'un milieu de culture liquide dans l'enveloppe (1) de réaction selon un débit contrôlé avec le moyen d'injection de liquide (71) ;
- injection d'un gaz dans l'enveloppe (1) de réaction selon un débit contrôlé avec le moyen d'injection de gaz (72) ;
- mise en circulation du milieu de culture liquide avec le moyen de mise en circulation (6) ;
- contrôle du moyen de mise en circulation (6) et du moyen d'injection de gaz (72) pour établir dans l'enveloppe (1) de réaction un régime d'écoulement diphasique gaz / milieu de culture liquide, notamment du type écoulement stratifié ou écoulement à poches ou à bulles allongées.

12. Procédé selon la revendication 11, dans lequel l'étape de contrôle comprend une étape de contrôle de la vitesse de circulation du liquide dans l'enveloppe (1) de réaction entre 0,1 et 1,0 m/s, et une étape de contrôle de la vitesse de circulation du gaz dans l'enveloppe (1) de réaction entre 0,5 et 2,0 m/s.

**Patentansprüche**

1. Reaktionsbehälter (1) für einen photosynthetischen Reaktor (2), der für die Kultur von photosynthetischen Mikroorganismen, vor allem von Algen, geeignet ist, wobei der Reaktionsbehälter (1) dafür konzipiert ist, zum einen auf einem Gewässer zu treiben, und zum anderen einen zweiphasigen Strömungsweg Gas / flüssiges Kulturmedium zwischen einer ersten und einer zweiten Öffnung (11, 12) des Reaktionsbehälters (1) zu begrenzen, wobei der Reaktionsbehälter (1) zum einen eine obere Membran (31) und eine untere Membran (32) umfasst, die mindestens teilweise aus einem nachgiebigen, dichten und für die Lichtstrahlung durchlässigen Material ausgeführt sind, wobei die Membrane (31, 32) gemäß Verbindungslinien (41, 42), die angrenzende aufblasbare Zellen (33, 34) begrenzen, hermetisch miteinander verbunden sind, und zum anderen Verbindungsbögen (35, 36), die die Zellen (33, 34) zweierweise verbinden, um den Strömungsweg von im Allgemeinen sinusförmiger Form zu definieren, wobei eine der Zellen (33) mit der ersten Öffnung (11) in Fluidverbindung steht, und eine andere Zelle (34) mit der zweiten Öffnung (12) in Fluidverbindung steht, wobei die Verbindungsbögen (35, 36) vom aufblasbaren Typ sind und mindestens teilweise aus einem nachgiebigen und dichten Material ausgeführt sind, wobei der Reaktionsbehälter (1) **dadurch gekennzeichnet ist, dass** der Behälter (1) aus drei verschiedenen Abschnitten besteht, nämlich einem stromaufwärtigen Abschnitt (100), einem stromabwärtigen Abschnitt (101) und einen mittleren Abschnitt (102), der zwischen dem stromaufwärtigen und stromabwärtigen Abschnitt (100, 101) eingesetzt ist, wobei:

- der stromaufwärtige Abschnitt (100) aus einer Anordnung einer oberen Haut und einer unteren Haut besteht, die gemäß Verbindungslinien (41, 42, 47) hermetisch miteinander verbunden sind, um gleichzeitig stromaufwärtige Abschnitte (330, 340) der Zellen (33, 34) und stromaufwärtige Verbindungsbögen (35) zu begrenzen, wobei die zwei Öffnungen (11, 12) in diesem stromaufwärtigen Abschnitt (100) vorgesehen sind;
- der stromabwärtige Abschnitt (101) aus einer Anordnung einer oberen Haut und einer unteren Haut besteht, die gemäß Verbindungslinien (41, 42, 47) hermetisch miteinander verbunden sind, um gleichzeitig stromabwärtige Abschnitte (331, 341) der Zellen (33, 34) und stromabwärtige Verbindungsbögen (36) zu begrenzen; und
- der mittlere Abschnitt (102) aus einer Anordnung einer oberen Haut und einer unteren Haut besteht, die gemäß Verbindungslinien (41) hermetisch miteinander verbunden sind, um ausschließlich mittlere Abschnitte (332, 342) der Zellen (33, 34) zu begrenzen.

2. Behälter (1) gemäß Anspruch 1, wobei jede Zelle (33; 34) von zwei geraden Verbindungslinien (41) begrenzt wird, um einen geraden Abschnitt des Strömungsweges zu definieren, und jeder Verbindungsbogen (35; 36) mindestens außen von einer im Wesentlichen 180° gebogenen Verbindungslinie (42) begrenzt wird und zwei Enden (43) aufweist, die mit zwei geraden Verbindungslinien (41) verbunden sind, die zwei angrenzende Zellen (33, 34) begrenzen.

3. Behälter (1) gemäß Anspruch 2, wobei die Enden (43) von jeder gebogenen Verbindungslinie (42) und/oder die freien Enden (45, 46) der geraden Verbindungslinien (41), die im Inneren der entsprechenden Verbindungsbögen (35, 36) liegen, mit Verstärkungsmitteln, vor allem vom Typ Öse, Niet oder Bolzen ausgestattet sind, die in dichter Weise durch die zwei Membrane hindurchgehen.

4. Behälter (1) gemäß Ansprüchen 2 oder 3, wobei jede gebogene Verbindungslinie (42) entweder eine im Allgemeinen Halbkreisform, oder eine Form im Allgemeinen einer aus mehreren geraden Segmenten gebildeten gebrochenen Linie aufweist.

5. Photosynthetischer Reaktor (2), der für die Kultur von photosynthetischen Mikroorganismen, vor allem von Algen, geeignet ist, umfassend:

- mindestens einen Reaktionsbehälter (1) nach einem der vorstehenden Ansprüche;
- mindestens eine Verschlussrohrleitung (5), die die Fluidverbindung zwischen der ersten und zweiten Öffnung (11, 12) des Reaktionsbehälters (1) sicherstellt;
- mindestens ein Umwälzmittel (6), das in der Verschlussrohrleitung (5) angeordnet und dafür konzipiert ist, das flüssige Kulturmedium in der Verschlussrohrleitung (5) und im Reaktionsbehälter (1) umzuwälzen;
- mindestens ein Flüssigkeits-Einspritzmittel (71), das in der Verschlussrohrleitung (5) angeordnet und dafür konzipiert ist, das Einspritzen der Flüssigkeit in den Reaktionsbehälter (1) zu ermöglichen;
- mindestens ein Gas-Einspritzmittel (72), das in der Verschlussrohrleitung (5) angeordnet und dafür konzipiert ist, das Einspritzen des Gases in den Reaktionsbehälter (1) zu ermöglichen;
- mindestens ein Flüssigkeits-Auslassmittel (81), um die Kultur photosynthetischer Mikroorganismen zu ernten; und
- mindestens ein Gas-Ablassmittel (82), das in der Verschlussrohrleitung (5) angeordnet und dafür konzipiert ist, das Ablassen des in den Reaktionsbehälter (1) eingespritzten Gases zu ermöglichen.

6. Photosynthetischer Reaktor (2) gemäß Anspruch 5, wobei die Verschlussrohrleitung (5) eine im Allgemeinen "U"- oder "V"-Form aufweist und umfasst:

   - einen aufsteigenden Abschnitt (51), der mit einem oberen Teil (510) versehen ist, der mit der ersten Öffnung (11) in Fluidverbindung steht; und
   - einen absteigenden Abschnitt (52), der mit einem oberen Teil (520) versehen ist, der mit der zweiten Öffnung (12) in Fluidverbindung steht;

   wobei der absteigende Abschnitt (52) und der aufsteigende Abschnitt (51) mit jeweiligen unteren Teilen (511, 521) versehen sind, die in Fluidverbindung stehen, und das Umwälzmittel (6) dafür konzipiert ist, das flüssige Kulturmedium im Reaktionsbehälter (1) von der ersten Öffnung (11) zur zweiten Öffnung (12) hin umzuwälzen.

7. Photosynthetischer Reaktor (2) gemäß Anspruch 6, wobei das Flüssigkeits-Einspritzmittel (71) im oberen Teil (510) des aufsteigenden Abschnitts (51) angeordnet ist, und das Gas-Einspritzmittel (72) im aufsteigenden Abschnitt (51), im oberen Teil (510) oder im unteren Teil (511) des aufsteigenden Abschnitts (51) angeordnet ist und wobei:

   - entweder das Gas-Ablassmittel (82) und das Flüssigkeits-Auslassmittel (81) aus einer gemeinsamen Leitung bestehen, die im oberen Teil (520) des absteigenden Abschnitts (52) angeordnet ist, wobei die gemeinsame Leitung mit einem freien Ende versehen ist, das auf einer in Bezug auf das Gewässer verstellbaren Höhe eingesetzt ist;
   - oder das Gas-Ablassmittel (82) aus einer ersten Auslassleitung besteht, die im oberen Teil (520) des absteigenden Abschnitts (52) angeordnet ist, wobei die Auslassmündung zur ersten Auslassleitung (81) hin gegebenenfalls mit einem Verschluss mit Schwimmer versehen ist, und das Flüssigkeits-Auslassmittel (81) aus einer zweiten Auslassleitung besteht, die im unteren Teil (511; 521) des einen oder anderen aus dem aufsteigenden (51) und absteigenden Abschnitt (52) angeordnet und mit einem freien Ende versehen ist, das auf einer in Bezug auf das Gewässer verstellbaren Höhe eingesetzt ist.

8. Photosynthetischer Reaktor (2) gemäß Anspruch 6, wobei das Flüssigkeits-Einspritzmittel (71) im oberen Teil (510) des aufsteigenden Abschnitts (51) angeordnet ist, und das Gas-Einspritzmittel (72) im absteigenden Abschnitt (52), vorzugsweise im oberen Teil (520) des absteigenden Abschnitts (52) angeordnet ist, und wobei das Gas-Ablassmittel (82) aus einer ersten Auslassleitung besteht, die im oberen Teil (510) des aufsteigenden Abschnitts (51) angeordnet ist, und das Flüssigkeits-Auslassmittel (81) aus einer zweiten Auslassleitung besteht, die entweder im oberen Teil (520) des absteigenden Abschnitts (52), oder im unteren Teil (511; 521) des einen oder anderen aus dem aufsteigenden (51) und absteigenden Abschnitt (52) angeordnet ist, wobei die zweite Auslassleitung mit einem freien Ende versehen ist, das auf einer in Bezug auf das Gewässer verstellbaren Höhe eingesetzt ist.

9. Photosynthetischer Reaktor (2) gemäß Anspruch 7, wobei das Umwälzmittel (6) aus einer Gas-Aufstiegsvorrichtung besteht und eine Gas-Einspritzleitung umfasst, die im unteren Teil (511, 521) des aufsteigenden Abschnitts (51) angeordnet ist, wobei die Gas-Einspritzleitung ebenfalls das Gas-Einspritzmittel (72) darstellt.

10. Photosynthetischer Reaktor (2) gemäß einem der Ansprüche 6 bis 8, wobei das Umwälzmittel (6) aus einer mechanischen Umwälzvorrichtung besteht, vor allem vom Typ einer Schraube (62), die von einem Motor (63) drehend angetrieben wird, oder einer Kreiselpumpe (65, 66), wobei die mechanische Umwälzvorrichtung im aufsteigenden Abschnitt (51) angeordnet ist.

**11.** Verfahren zum Kultivieren von photosynthetischen Mikroorganismen, vor allem von Algen, unter Verwendung eines Reaktors (2) nach einem der Ansprüche 5 bis 10 und die folgenden Schritte umfassend:

> - Einspritzen eines flüssigen Kulturmediums in den Reaktionsbehälter (1) gemäß einer gesteuerten Rate mit dem Flüssigkeits-Einspritzmittel (71);
> - Einspritzen eines Gases in den Reaktionsbehälter (1) gemäß einer gesteuerten Rate mit dem Gas-Einspritzmittel (72);
> - Umwälzen des flüssigen Kulturmediums mit dem Umwälzmittel (6);
> - Steuern des Umwälzmittels (6) und des Gas-Einspritzmittels (72), um im Reaktionsbehälter (1) ein zweiphasiges Strömungsregime Gas / flüssiges Kulturmedium, vor allem vom Typ geschichtete Strömung oder Strömung mit länglichen Taschen oder Blasen herzustellen.

**12.** Verfahren gemäß Anspruch 11, wobei der Schritt des Steuerns einen Schritt des Steuerns der Umwälzgeschwindigkeit der Flüssigkeit im Reaktionsbehälter (1) zwischen 0,1 und 1,0 m/s, und einen Schritt des Steuerns der Umwälzgeschwindigkeit des Gases im Reaktionsbehälter (1) zwischen 0,5 und 2,0 m/s umfasst.

**Claims**

**1.** A reaction casing (1) for a photosynthetic reactor (2) adapted for the culture of photosynthetic microorganisms, in particular algae, said reaction casing (1) being designed, on one hand, to float on an expanse of water and, on the other hand, to delimit a gas/liquid culture medium two-phase flow path between a first and a second openings (11, 12) of said reaction casing (1), wherein said reaction casing (1) includes, on one hand, an upper membrane (31) and a lower membrane (32) made at least partially of a flexible and watertight material transparent to light radiation, said membranes (31, 32) being hermetically bonded together along junction lines (41, 42) delimiting adjacent inflatable cells (33, 34) and, on the other hand, junction elbows (35, 36) pairwise joining said cells (33, 34) in order to define said flow path of a generally sinuous shape, one of the cells (33) being in fluid connection with the first opening (11) and another cell (34) being in fluid connection with the second opening (12), where the junction elbows (35, 36) are of the inflatable type and are made at least partially of a flexible and watertight material, said reaction casing (1) being **characterized in that** the casing (1) is composed of three distinct portions, namely an upstream portion (100), a downstream portion (101) and a central portion (102) interposed between the upstream and downstream portions (100, 101), wherein:

> - the upstream portion (100) is composed of an assembly of an upper skin and of a lower skin hermetically bonded together along junction lines (41, 42, 47) to delimit both upstream portions (330, 340) of the cells (33, 34) and upstream junction elbows (35), the two openings (11, 12) being provided in this upstream portion (100);
> - the downstream portion (101) is composed of an assembly of an upper skin and of a lower skin hermetically bonded together along junction lines (41, 42, 47) to delimit both downstream portions (331, 341) of the cells (33, 34) and downstream junction elbows (36); and
> - the central portion (102) is composed of an assembly of an upper skin and of a lower skin hermetically bonded together along junction lines (41) to delimit only central portions (332, 342) of the cells (33, 34).

**2.** The reaction casing (1) according to claim 1, wherein each cell (33; 34) is delimited by two rectilinear junction lines (41) to define a rectilinear portion of the flow path, and each junction elbow (35; 36) is delimited at least externally by a curved junction line (42) substantially bent at 180° and having two ends (43) linked to two rectilinear junction lines (41) delimiting two adjacent cells (33, 34).

**3.** The reaction casing (1) according to claim 2, wherein the ends (43) of each curved junction line (42) and/or the free ends (45, 46) of the rectilinear junction lines (41) located inside the corresponding junction elbows (35, 36) are provided with reinforcing means, in particular of the eyelet, rivet or bolt type hermetically crossing the two membranes.

**4.** The reaction casing (1) according to claim 2 or 3, wherein each curved junction line (42) has either a general shape of a semi-circle, or a general shape of a broken line formed of several rectilinear segments.

**5.** A photosynthetic reactor (2) adapted for cultivating photosynthetic microorganisms, in particular algae, including:

> - at least one reaction casing (1) in accordance with any one of the preceding claims;
> - at least one closing pipe (5) ensuring the fluid connection between the first and second openings (11, 12) of said reaction casing (1);
> - at least one circulating mean (6) disposed in said closing pipe (5) and designed to put into circulation the liquid culture medium in the closing pipe (5) and in the reaction casing (1);
- at least one liquid injection mean (71) disposed in said closing pipe (5) and designed to allow injecting the liquid into the reaction casing (1);
- at least one gas injection mean (72) disposed in said closing pipe (5) and designed to allow injecting gas into the reaction casing (1),
- at least one liquid outlet mean (81) to harvest the culture of photosynthetic microorganisms; and
- at least one gas exhaust mean (82) disposed in said closing pipe (5) and designed to allow the exhaust of the gas injected into the reaction casing (1).

6. The photosynthetic reactor (2) according to claim 5, wherein the closing pipe (5) has a general « U » or « V » shape and comprises:

- an ascending portion (51) provided with a top part (510) in fluid connection with the first opening (11); and
- a descending portion (52) provided with a top part (520) in fluid connection with the second opening (12),

the descending portion (52) and the ascending portion (51) being provided with respective bottom parts (511, 521) in fluid connection, and the circulating mean (6) being for putting the liquid culture medium in circulation into the reaction casing (1) from the first opening (11) to the second opening (12).

7. The photosynthetic reactor (2) according to claim 6, wherein the liquid injection mean (71) is disposed in the top part (510) of the ascending portion (51), and the gas injection mean (72) is disposed in the ascending portion (51), in the top part (510) or in the bottom part (511) of said ascending portion (51), and wherein:

- either the gas exhaust mean (82) and the liquid outlet mean (81) are constituted of a common conduit disposed in the top part (520) of the descending portion (52), said common conduit being provided with a free end set at an adjustable height with respect to the expanse of water;
- or the gas exhaust mean (82) is constituted of a first outlet conduit disposed in the top part (520) of the descending portion (52), the outlet orifice to said first outlet conduit (81) being possibly provided with a float shutoff, and the liquid outlet mean (81) is constituted of a second outlet conduit disposed in the bottom part (511; 521) of either one of the ascending (51) and descending (52) portions and provided with a free end set at an adjustable height with respect to the expanse of water.

8. The photosynthetic reactor (2) according to claim 6, wherein the liquid injection mean (71) is disposed in the top part (510) of the ascending portion (51), and the gas injection mean (72) is disposed in the descending portion (52), preferably in the top part (520) of said descending portion (52), and wherein the gas exhaust mean (82) is constituted of a first outlet conduit disposed in the top part (510) of the ascending portion (51) and the liquid outlet mean (81) is constituted of a second outlet conduit disposed either in the top part (520) of the descending portion (52), or in the bottom part (511; 521) of either one of the ascending (51) and descending (52) portions, said second outlet conduit being provided with a free end set at an adjustable height with respect to the expanse of water.

9. The photosynthetic reactor (2) according to claim 7, wherein the circulating mean (6) is constituted of a gas lift device and comprises a gas injection conduit disposed in the bottom part (511, 521) of the ascending portion (51), said gas injection conduit also constituting the gas injection mean (72).

10. The photosynthetic reactor (2) according to any one of claims 6 to 8, wherein the circulating mean (6) is constituted of a mechanical circulating device, in particular of the type propeller (62) driven in rotation by a motor (63) or centrifugal pump (65, 66), said mechanical circulating device being disposed in the ascending portion (51).

11. A method for cultivating photosynthetic microorganisms, in particular algae, using a reactor (2) according to any one of claims 5 to 10 and comprising the following steps:

- injecting a liquid culture medium into the reaction casing (1) according to a flow rate controlled with the liquid injection mean (71);
- injecting a gas into the reaction casing (1) according to a flow rate controlled with the gas injection mean (72);
- circulating the liquid culture medium with the circulating mean (6);
- controlling the circulating mean (6) and the gas injection mean (72) for establishing in the reaction casing (1) a gas/liquid culture medium two-phase flow regime, in particular of the type laminated flow or elongated bubble or pocket flow.

12. The method according to claim 11, wherein the control step comprises a step of controlling the circulation velocity of the liquid in the reaction casing (1) between 0.1 and 1.0 m/s, and a step of controlling the circulation velocity of the gas in the reaction casing (1) between 0.5 and 2.0 m/s.

Fig. 1

Fig. 2

Fig. 3

**Fig. 4a**

**Fig. 4b**

**Fig. 5a**

*Fig. 5b*

*Fig. 5c*

*Fig. 6a*

*Fig. 6b*

*Fig. 6c*

*Fig. 7a*

*Fig. 7b*

*Fig. 7c*

*Fig. 8*

*Fig. 9a*

*Fig. 9b*

*Fig. 9c*

Fig. 10

Fig. 11a

Fig. 11b

Fig. 12

EP 2 780 442 B1

**Fig. 13a**

**Fig. 13b**

Fig. 14a

Fig. 14b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- GB 2118572 A
- ES 2193860 A1
- GB 2331762 A
- ES 2150389 A1
- FR 2685344 A1
- FR 2875511 A3
- FR 2621323
- FR 2361060

- FR 2324224
- WO 2009051479 A2
- WO 2008134010 A2
- WO 2009090549 A2
- GB 2473865 A
- WO 2010012028 A
- US 3955317 A